# EUROPEAN PATENT APPLICATION

(11) **EP 4 353 733 A2**
(43) Date of publication of application: **17.04.2024**
(21) Application number: 22820464.0
(22) Date of filing: 31.05.2022
(51) Int. Cl.: C07H 19/167, A61K 31/7064, A61P 35/00, C07D 487/04

(54) **NUCLEOSIDE DERIVATIVE HAVING MULTI-TARGET KINASE INHIBITORY ACTIVITY AND PHARMACEUTICAL COMPOSITION FOR PREVENTING AND TREATING CANCER COMPRISING SAME**

(30) Priority: 08.06.2021 KR 20210073914
(71) Applicant: Future Medicine Co., Ltd., Seongnam-si, Gyeonggi-do 13449 (KR)
(72) Inventor: JEONG, Lak Shin, Seoul 06216 (KR); LEE, Sang Kook, Seoul 06288 (KR); TRIPATHI, Sushil Kumar, Seoul 08826 (KR); MASHELKAR, Karishma K., Seoul 08818 (KR); SUNG, Ki Su, Seoul 08824 (KR); YUM, Yun A, Seoul 05673 (KR); KWON, Jee Youn, Seoul 08833 (KR)
(74) Representative: Roos, Peter
(86) International application number: PCT/KR2022/007747
(87) International publication number: WO 2022/260336

(57) **Abstract**

The present invention relates to a nucleoside derivative, which can be used for the prevention or treatment of cancer by inhibiting a kinase, and a pharmaceutical composition including the same, wherein the nucleoside derivative has inhibitory activity against kinases such as NTRK1, CSNK1D, DYRK1A, DYRK1B, and FLT3, and thus, exhibits inhibitory activity against various cancer cells directly or indirectly associated with the kinase, so that the nucleoside derivative can be used to prevent or treat various cancers such as lung cancer, colon cancer, breast cancer, liver cancer, stomach cancer, and prostate cancer.

## Description

### [Technical Field]

The present invention relates to a nucleoside derivative, which can be used for the prevention or treatment of cancer by inhibiting a kinase, and a pharmaceutical composition including the same.

### [Background Art]

One of the main causes of cancer growth is the mutation of kinases. Protein kinases play an important role in cellular functions by mediating the phosphorylation of proteins. Since they transfer the terminal phosphate group of adenosine triphosphate (ATP) to protein substrates to initiate reactions such as signal transduction, gene regulation, and metabolism, dysregulation of kinases may be associated with various diseases including cancer. Therefore, when a nucleoside derivative is developed by targeting a kinase, the nucleoside derivative may be used for cancer treatment.

A major problem with anticancer drugs is drug resistance, which often occurs in cancer patients receiving a monotherapy. In such situations, multiple drug therapy strategies may overcome the resistance crisis. Multi-target inhibitors that target one or more targets may surpass the effects of single-target inhibitors by preventing the proliferation of cancer cells and, secondly, preventing the microenvironment that supports tumor development.

Meanwhile, the nitrogenous base of ATP interacts with a hinge region through hydrogen bonds and a ribose ring interacts with the polar residues of a sugar pocket, whereas known kinase inhibitors occupy the hinge region or a hydrophobic region without binding to the sugar pocket. Therefore, the present inventors recognized that in the design of a selective kinase inhibitor, a hydrophobic pocket to which ATP does not bind is an important site for the development of the inhibitor.

Accordingly, the present inventors designed a nucleoside kinase inhibitor occupying hydrophobic pockets by allowing the nitrogenous base to form hydrogen bonds with the hinge region and modifying a hydrophobic residue (R₁ of the following Chemical Formula 1) and another hydrophobic residue (R₂ of the following Chemical Formula 1). Further, the present inventors found that the polar hydroxyl groups of a sugar, such as the ribose ring of ATP, form hydrogen bonds with sugar regions, allowing the molecules to fit together, which increases binding strength. In addition, based on the fact that by modifying a part of the sugar (Y of the following Chemical Formula 1), this part forms an important bond with the triphosphate binding site of a kinase domain, the kinase inhibitory activity of synthesized compounds was evaluated. Through this, the present inventors searched for selective inhibitors which are effective against NTRK1, DYRK1A, DYRK1B, FLT3, and CSNK1D, which are kinases associated with overexpression in cancer cells, and confirmed that the inhibitors exhibit excellent anticancer activity, thereby completing the present invention.

### [Disclosure]

### [Technical Problem]

Accordingly, a problem to be solved by the present invention is to provide a nucleoside derivative, which can be used for the prevention or treatment of cancer by selectively inhibiting a specific kinase, and a pharmaceutical composition including the same.

### [Technical Solution]

A nucleoside derivative according to an embodiment of the present invention for solving the problem is represented by the following Chemical Formula 1. 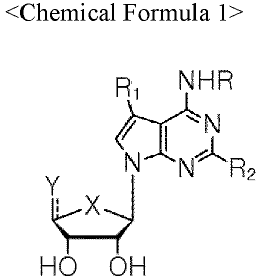
X is oxygen (O) or sulfur (S),
R is hydrogen (H); a substituted or unsubstituted C₁ to C₁₀ alkyl; or a substituted or unsubstituted C₆ to C₂₀ aryl or alkylaryl,
R₁ is a substituted or unsubstituted C₂ to C₁₀ heteroaryl; a substituted or unsubstituted C₂ to C₈ alkenyl; a substituted or unsubstituted C₆ to C₂₀ aryl; a substituted or unsubstituted C₂ to C₈ alkynyl; cyano; amide; or carboxyl,
R₂ is a halogen; substituted or unsubstituted amine; or a substituted or unsubstituted C₆ to C₂₀ aryl or alkylaryl, and
Y is hydrogen (H); an alkyl substituted with hydroxy, ester, alkoxy, aryl, heteroaryl, heterocycloalkyl, cycloalkyl, azide, amine, substituted amine, urea, or amide; or =CZ₂, and Z is each independently hydrogen (H) or a C₂ to C₈ alkyl.

When R, R₁, R₂ and Y are substituted, R, R₁, R₂ and Y may be substituted with one or more of a C₁ to C₆ alkyl, a C₂ to C₁₀ heteroaryl and sulfonamide.

The heteroaryl may be furanyl, thiophenyl, pyrrolyl, pyranyl, pyrazolyl, pyridinyl, triazolyl, imidazolyl, thiazolyl, pyrimidinyl, pyrazinyl, pyridazinyl, indolyl, quinolinyl or purinyl.

The heterocycloalkyl may be tetrahydrofuranyl, thiolanyl, pyrrolidinyl, tetrahydropyranyl, piperidinyl, dioxanyl, morpholino or tetrahydropyrimidinyl.

The aryl may be phenyl, naphthalenyl, anthracenyl or phenanthrenyl, and the alkylaryl may be benzyl.

The alkynyl may be ethynyl, propynyl, butynyl, pentynyl, hexynyl, heptynyl, octynyl, nonynyl or decynyl.

The alkenyl may be ethenyl, propenyl, butenyl, pentenyl, hexenyl, heptenyl, octenyl, nonenyl or decenyl.

The alkyl may be methyl, ethyl, propyl, butyl or pentyl.

Specifically, X may be sulfur, R may be hydrogen, R₁ may be furanyl, thiophenyl, vinyl, phenyl, thiomorpholino phenyl, thiomorpholino dioxide phenyl, sulfonamide phenyl, ethylsulfonamide phenyl, ethynyl, propynyl, butynyl, dimethylbutynyl, cyclopropylethynyl, cyano, amide or carboxy, R₂ may be chlorine (Cl) or amine (-NH₂), and Y may be hydrogen, - CH₂OH, -CH₂OCOAr, -CH₂N₃, -CH₂NH₂, -CH₂NHCOAr, -CH₂NHCONH₂, -CH₂N-alkyl, - CH₂N-cycloalkyl, -CH₂N(CH₂CH₂)₂O or =CH₂.

In this case, the 'Ar' may be phenyl, chlorophenyl, methoxyphenyl, dimethylphenyl or nicotinyl as an aryl, the 'alkyl' may be methyl, ethyl, propyl, butyl or pentyl, and the 'cycloalkyl' may be cyclopropyl, cyclobutyl, cyclopentyl or cyclohexyl.

More specifically, the nucleoside derivative according to an embodiment of the present invention may be represented by one of the following Chemical Formulae 1a and 1b, 2a to 2d, 3, 4a to 4d, 5, 6, 7a to 7h, 8a to 8e, 9a to 9c, 10a to 10c, 11ai to 11aiii, 11b, 12a and 12b, 13a to 13g and 14.

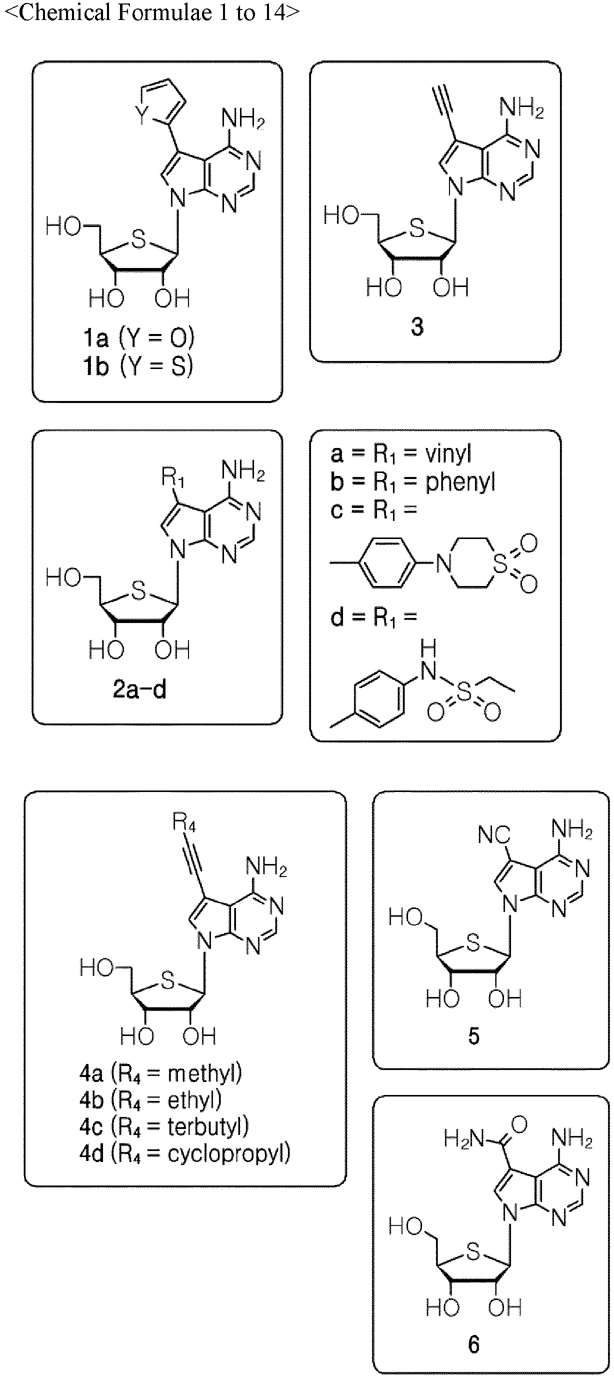

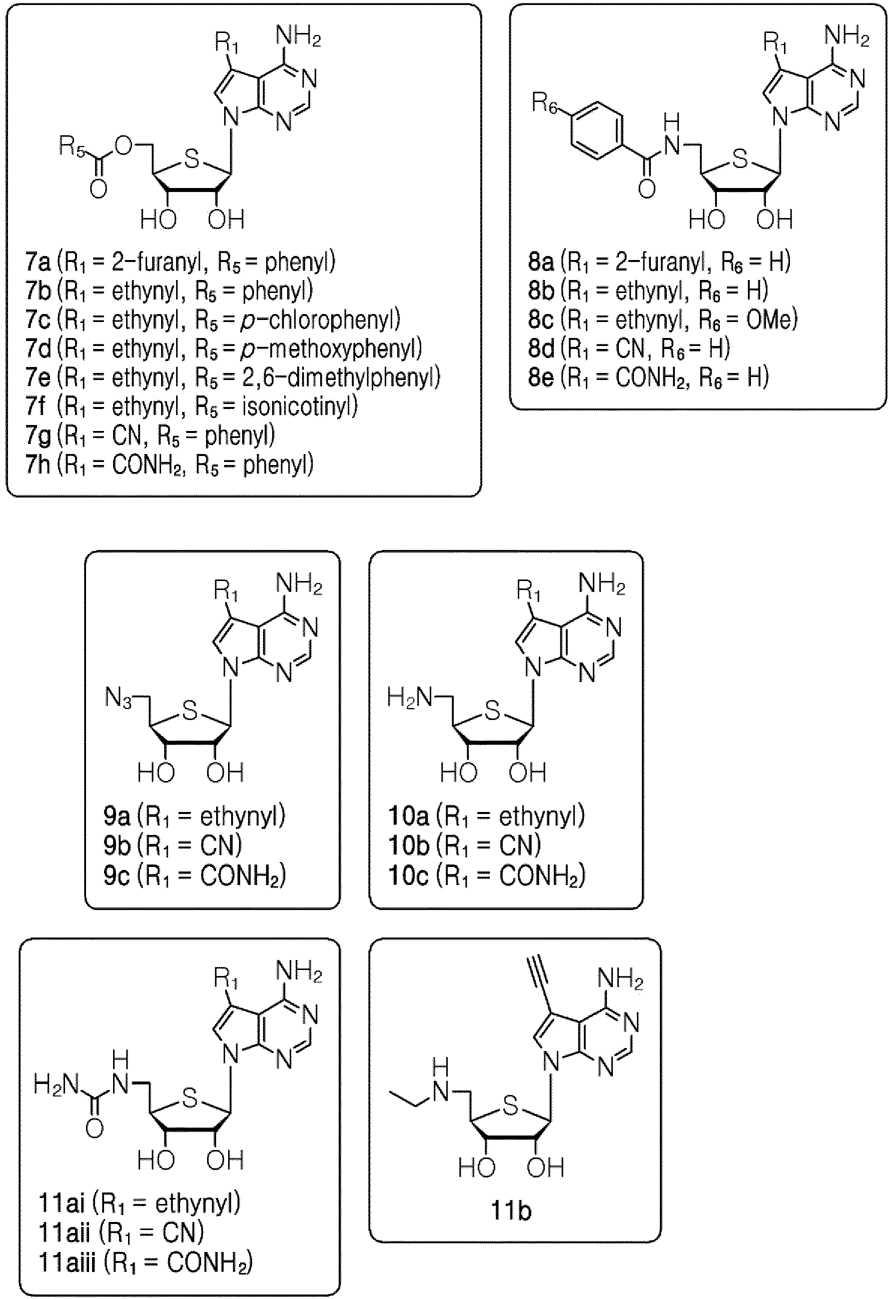

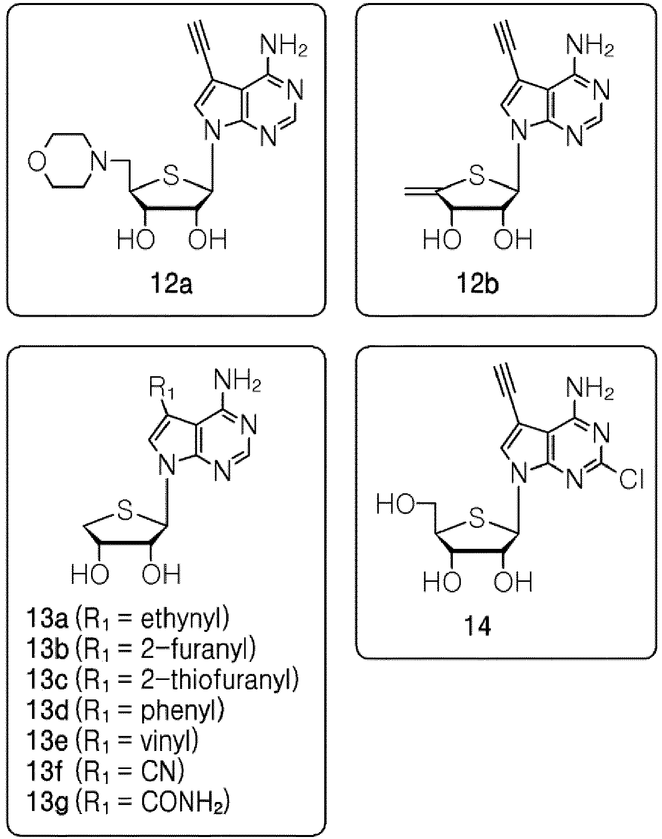

The nucleoside derivative of the present invention may be provided in the form of a pharmaceutically acceptable salt. As a salt, acid addition salts formed by various organic or inorganic acids, which are pharmaceutically acceptable, are useful.

However, the salt is not limited thereto, and the nucleoside derivative of the present invention may also be provided in the form of all salts, hydrates and solvates which may be prepared by typical methods.

The nucleoside derivative of the present invention as described above has inhibitory activity against kinases such as NTRK1, CSNK1D, DYRK1A, DYRK1B and FLT3 (see Experimental Example 1), and thus, exhibits inhibitory activity against various cancer cells directly or indirectly associated with the kinase (see Experimental Example 2), so that the nucleoside derivative may be included in a pharmaceutical composition capable of preventing or treating various cancers such as lung cancer, colon cancer, breast cancer, liver cancer, stomach cancer and prostate cancer.

That is, the pharmaceutical composition for preventing or treating cancer of the present invention may include the nucleoside derivative or a pharmaceutically acceptable salt thereof, the cancer may be one or more selected from the group consisting of lung cancer, colon cancer, breast cancer, liver cancer, stomach cancer and prostate cancer, and the nucleoside derivative or the pharmaceutically acceptable salt thereof may have kinase inhibitory activity.

The pharmaceutical composition may be administered systemically or locally, and may be formulated using an excipient (or a diluent) such as a filler, an extender, a binder, a wetting agent, a disintegrating agent, and a surfactant, which may be generally used for administration.

A preferred dose of the pharmaceutical composition varies depending on various factors such as the condition and body weight of a patient, the degree of a disease, the form of drug, the administration route, and the duration, but may be appropriately selected by the person skilled in the art. Further, the administration route may be changed depending on the condition of a patient and the severity thereof.

Specific details of other embodiments are included in the detailed description.

### [Advantageous Effects]

The nucleoside derivative according to the embodiments of the present invention or a pharmaceutically acceptable salt thereof has inhibitory activity against kinases such as NTRK1, CSNK1D, DYRK1A, DYRK1B and FLT3, and thus, exhibits inhibitory activity against various cancer cells directly or indirectly associated with the kinase, so that the nucleoside derivative or the pharmaceutically acceptable salt thereof can be used to prevent or treat various cancers such as lung cancer, colon cancer, breast cancer, liver cancer, stomach cancer and prostate cancer.

### [Modes of the Invention]

The terms used in the present specification are used merely to describe embodiments, and are not intended to limit the present invention. In the present specification, the 'and/or' includes each and all combinations of one or more of the items mentioned. Further, the singular form also includes the plural forms unless specifically stated in a phrase. The terms 'comprises' and/or 'comprising' used in the specification do not exclude the presence or addition of one or more other constituent elements in addition to the referenced constituent elements. The numerical range indicated by using '-' or 'to' indicates a numerical range including values described before and after it as a lower limit and an upper limit, respectively, unless otherwise stated. 'About' or 'approximately' means a value or numerical range within 20% of the value or numerical range described thereafter.

As used herein, 'prevention' refers to suppressing the onset of a symptom or disease in an individual who does not yet have the symptom or disease, but may suffer from the symptom or disease.

As used herein, 'treatment' refers to (a) suppression of the development (exacerbation) of a symptom or disease, (b) reduction or amelioration of a symptom or disease, or (c) elimination of a symptom or disease, from an individual.

As used herein, 'individual' refers to an animal, particularly a mammal, including a human having a symptom or disease that can be 'prevented' or 'treated' by administering the composition of the present invention.

As used herein, 'substituted Cₙ to Cₙ₊ₘ' compound includes a case where the number of all carbons of a compound including a substituted moiety is n to n+m, as well as a case where the number of carbons of the compound except for the substituted moiety is n to n+m.

Hereinafter, embodiments of the present invention will be described in detail with reference to Preparation Examples and Experimental Examples, but it is obvious that the effects of the present invention are not limited by the following Experimental Examples.

### Preparation Examples: Preparation of nucleoside derivative of present invention

### Preparation Example 1: Synthesis of Compounds 1a and 1b and 2a to 2d

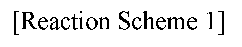

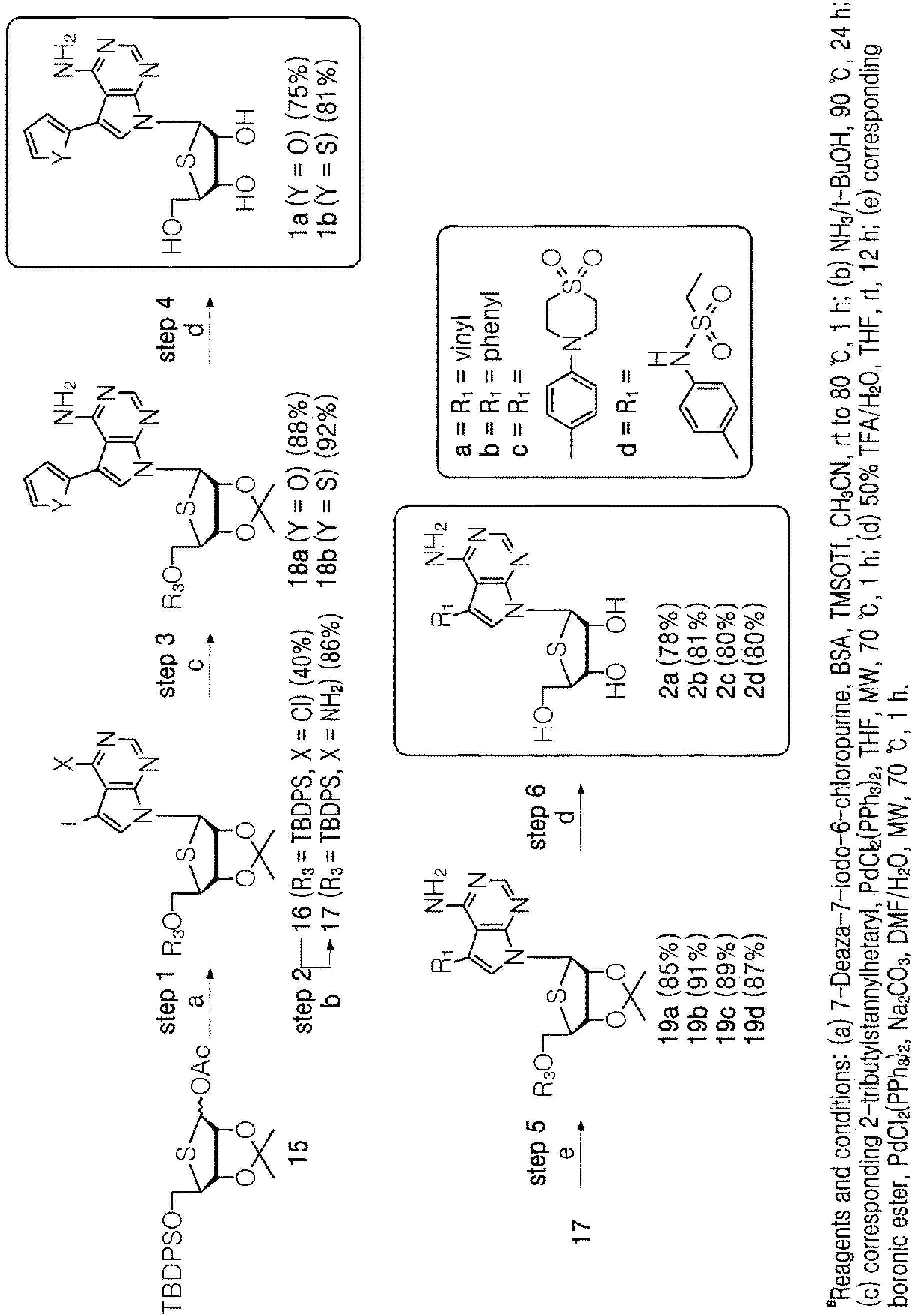

### Preparation Example 1-1: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-4-chloro-5-iodo-7H-pyrrolo[2,3-d]pyrimidine (Compound 16)

4-chloro-5-iodo-7H-pyrrolo[2,3-d]pyrimidine (2.6 g, 9.33 mmol) and bis(trimethylsilyl)acetamide (BSA, 2.5 mL, 10.27 mmol) were added to anhydrous acetonitrile under nitrogen substitution, and the resulting mixture was stirred for 10 minutes. Compound 15 (5 g, 10.72 mmol) was dissolved in anhydrous acetonitrile, and then added to this solution, trimethylsilyl trifluoromethanesulfonate (1.5 mL, 8.40 mmol) was added dropwise thereto, and the resulting mixture was stirred at room temperature for 15 minutes, and then further stirred at 80°C for 1 hour. After it was confirmed that the reaction was terminated, the resulting product was extracted with ethyl acetate (700 mL) at room temperature, and the organic layer was partitioned, washed with a saturated aqueous sodium bicarbonate solution and brine, and dried over anhydrous magnesium sulfate. The remaining solid was removed by performing filtration under reduced pressure, the residue was concentrated under reduced pressure, and then subjected to silica gel column chromatography to obtain Compound 16 (2.9 g, 40%).

¹H NMR (CD₃OD, 500 MHz): *δ* 8.46 (s, 1H), 7.87 (s, 1H), 7.64-7.58 (m, 4 H), 7.40-7.33 (m, 4H), 7.31-7.28 (m, 2H), 6.24 (d, *J* = 2.2 Hz, 1H), 5.10 (dd, *J* = 5.5, 2.2 Hz, 1H), 4.96 (dd, *J* = 5.5, 2.1 Hz, 1H), 3.88 (dd, *J* = 10.4, 7.2 Hz, 1H), 3.81 (dd, *J* = 10.4, 7.3 Hz, 1H), 3.75 (td, *J* = 7.2, 2.1 Hz, 1H), 1.55 (s, 3H), 1.28 (s, 3H), 1.04 (s, 9H); HRMS (ESI-Q-TOF) *m*/*z* [M + H]⁺ for C₃₀H₃₄ClIN₃O₃SSi calculated 706.0818, found 706.0798.

### Preparation Example 1-2: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-5-iodo-7H-pyrrolo[2,3-d]pyrimidine-4-amine (Compound 17)

After the material of Compound 16 (2.9 g, 4.11 mmol) was dissolved in NH₃/*t*-BuOH (30 mL) in a stainless steel bomb, the resulting solution was stirred at 90°C for 24 hours. After the reaction was terminated, the reaction solution was concentrated under reduced pressure and separated by silica gel column chromatography to obtain Compound 17 (2.42 g, 86%).

¹H NMR (CDCl₃, 500 MHz): *δ* 8.22 (s, 1H), 7.64-7.62 (m, 4H), 7.43-7.40 (m, 2H), 7.37-7.33 (m, 5H), 6.21 (d, *J* = 2.7 Hz, 1H), 5.88 (brs, 2H), 4.88 (dd, *J* = 5.6, 2.8 Hz, 1H), 4.78 (dd, *J* = 5.6, 2.8 Hz, 1H), 3.86-3.79 (m, 2H), 3.77-3.75 (m, 1H), 1.58 (s, 3H), 1.27 (s, 3H), 1.07 (s, 9H); HRMS (ESI-Q-TOF) *m*/*z* [M + H]⁺ for C₃₀H₃₆IN₄O₃SSi calculated 687.1317, found 687.1301.

### Preparation Example 1-3: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-5-(furan-2-yl)-7H-pyrrolo[2,3-d]pyrimidine-4-amine and 7-((3aR,4R,6R,6aS)-6-(((tert-butyldiphenylsilyl)oxy)methyl)-2,2-dimethyltetrahydrothieno[3,4-d][1,3]dioxol-4-yl)-5-(thiophen-2-yl)-7H-pyrrolo[2,3-d]pyrimidine-4-amine (Compounds 18a/18b)

Known synthetic method (A): After Compound 17 (1 eq.) was dissolved in tetrahydrofuran, a PdCl₂(PPh₃)₂ (15 mol%) catalyst was added and the resulting mixture was substituted with nitrogen for 5 minutes. After 2-(tributylstannyl)hetaryl (2.5 eq.) was added thereto, the resulting mixture was stirred at 70°C in a microwave for 1 hour. After the reaction was completed, the reaction was terminated with water and brine, and extraction was performed with ethyl acetate. The organic solvent layer was partitioned, concentrated under reduced pressure, and then subjected to column chromatography to separate a pure material.

Compound 18a was obtained as a yellow sticky material in a yield of 88%; ¹H NMR (CDCl₃, 400 MHz): *δ* 8.27 (s, 1H), 7.64-7.61 (m, 4H), 7.47-7.46 (m, 1H), 7.39-7.36 (m, 3H), 7.33-7.29 (m, 4H), 6.45-6.44 (m, 1H), 6.28-6.27 (m, 2H), 5.98 (brs, 2H), 4.95 (dd, *J* = 5.9, 3.2 Hz, 1H), 4.82 (dd, *J* = 5.9, 3.2 Hz, 1H), 3.92-3.82 (m, 2H), 3.79-3.75 (m, 1H), 1.59 (s, 3H), 1.28 (s, 3H), 1.05 (s, 9H); HRMS (ESI-Q-TOF) *m*/*z* [M + H]⁺ for C₃₄H₃₉N₄O₄SSi calculated 627.2456, found 627.2463.

Compound 18b was obtained as a colorless sticky material in a yield of 92%; ¹H NMR (CDCl₃, 400 MHz): *δ* 8.28 (s, 1H), 7.62-7.59 (m, 4H), 7.37-7.34 (m, 3H), 7.32-7.27 (m, 4H), 7.23 (s, 1H), 7.10-7.07 (m, 1H), 7.00-6.99 (m, 1H), 6.28 (d, *J* = 2.7 Hz, 1H), 5.41 (brs, 2H), 4.94 (dd, *J* = 5.5, 3.2 Hz, 1H), 4.81 (dd, *J* = 5.9, 3.2 Hz, 1H), 3.91-3.82 (m, 2H), 3.79-3.74 (m, 1H), 1.59 (s, 3H), 1.28 (s, 3H), 1.03 (s, 9H); HRMS (ESI-Q-TOF) *m*/*z* [M + H]⁺ for C₃₄H₃₉N₄O₃S₂Si calculated 643.2227, found 643.2218.

### Preparation Example 1-4: Synthesis of (2R,3R,4S,5R)-2-(4-amino-5-(furan-2-yl)-7H-pyrrolo[2,3-d]pyrimidin-7-yl)-5-(hydroxymethyl)tetrahydrothiophene-3,4-diol and (2R,3R,4S,5R)-2-(4-amino-5-(thiophen-2-yl)-7H-pyrrolo[2,3-d]pyrimidin-7-yl)-5-(hydroxymethyl)tetrahydrothiophene-3,4-diol (Compounds 1a and 1b)

Known synthetic method (B): After Compounds 18a/18b were dissolved in tetrahydrofuran, a 50% aqueous trifluoroacetic acid solution was added dropwise thereto, and the resulting mixture was stirred for 12 hours. The mixture was neutralized by stirring with a weakly basic cation exchange resin (Dowex^{®} 66 free base) for 1 hour, and the remaining solid was removed by performing filtration under reduced pressure. After concentration under reduced pressure, Compounds 1a/1b were obtained in a yield of 75% and 81%, respectively, through silica gel column chromatography.

### Preparation Example 1-5: Synthesis of Compounds 19a to 19d

Known synthetic method (C): Borate ester, PdCl₂(PPh)₃ (6 mol%), and sodium carbonate (2 eq.) were added to Compound 17 (1 eq.), and the resulting mixture was dissolved in nitrogen-substituted dimethylformamide/distilled water (0.14 M/0.36 M), and then stirred at 70°C in a microwave for 1 hour. After the reaction was terminated with distilled water, extraction was performed with ethyl acetate. The organic solvent layer was partitioned, and then washed with distilled water and brine, concentrated under reduced pressure, and then subjected to silica gel column chromatography to obtain Compounds 19a to 19d.

7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-5-vinyl-7*H*-pyrrolo[2,3-*d*]pyrimidine-4-amine (Compound 19a). Compound 19a was obtained as a yellow sticky material in a yield of 85%; ¹H NMR (CD₃OD, 400 MHz): *δ* 8.03 (s, 1H), 7.64 (t, *J* = 9.2 Hz, 4H), 7.42-7.39 (m, 3 H), 7.37-7.31 (m, 4H), 6.91 (dd, *J* = 17.2, 10.8 Hz, 1H), 6.21 (s, 1H), 5.44 (d, *J* = 17.6 Hz, 1H), 5.20 (d, *J* = 10.8 Hz, 1H), 5.07-5.05 (m, 1H), 4.96-4.95 (m, 1H), 3.93-3.89 (m, 1H), 3.86-3.81 (m, 1H), 3.73-3.69 (m, 1H), 1.55 (s, 3H), 1.29 (s, 3H), 1.04 (s, 9H); HRMS (ESI-Q-TOF) *m*/*z* [M + H]⁺ for C₃₂H₃₉N₄O₃SSi calculated 587.2507, found 587.2506.

7-((3a*R*,*4R,6R,6aS*)-6-(((*tert*-butyldiphenylsilyl)oxy)methyl)-2,2-dimethyltetrahydrothieno[3,4-*d*][1,3]dioxol-4-yl)-5-phenyl-7*H*-pyrrolo[2,3-*d*]pyrimidine-4-amine (Compound 19b). Compound 19b was obtained as a colorless sticky material in a yield of 91%; ¹H NMR (CD₃OD, 400 MHz): *δ* 8.11 (s, 1H), 7.62-7.59 (m, 4H), 7.43-7.41 (m, 3H), 7.39-7.33 (m, 4H), 7.29-7.26 (m, 4H), 7.23 (s, 1H), 6.27 (d, *J* = 2.7 Hz, 1H), 5.09 (dd, *J* = 5.4, 2.7 Hz, 1H), 4.96 (dd, *J* = 5.5, 2.3 Hz, 1H), 3.92-3.82 (m, 2H), 3.75-3.71 (m, 1H), 1.57 (s, 3H), 1.30 (s, 3H), 1.01 (s, 9H); HRMS (ESI-Q-TOF) *m*/*z* [M + H]⁺ for C₃₆H₄₁N₄O₃SSi calculated 637.2663, found 637.2646.

4-(4-(4-amino-7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-7*H*-pyrrolo[2,3-*d*]pyrimidin-5-yl)phenyl)thiomorpholine 1,1-dioxide (Compound 19c). Compound 19c was obtained as a colorless sticky material in a yield of 89%; ¹H NMR (CDCl₃, 400 MHz): *δ* 8.27 (s, 1H), 7.61-7.59 (m, 4H), 7.40-7.34 (m, 4H), 7.32-7.27 (m, 4H), 7.14 (s, 1H), 6.94-6.92 (m, 2H), 6.31 (d, *J* = 3.6 Hz, 1H), 5.28 (brs, 2H), 4.99 (dd, *J* = 5.9, 3.2 Hz, 1H), 4.82 (dd, *J* = 5.9, 2.7 Hz, 1H), 3.93-3.86 (m, 4H), 3.84-3.82 (m, 2H), 3.79-3.75 (m, 1H), 3.13-3.11 (m, 4H), 1.60 (s, 3H), 1.29 (s, 3H), 1.03 (s, 9H); HRMS (ESI-Q-TOF) *m*/*z* [M + H]⁺ for C₄₀H₄₈N₅O₅S₂Si calculated 770.2861, found 770.2865.

*N*-(4-(4-amino-7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-7*H*-pyrrolo[2,3-*d*]pyrimidin-5-yl)phenyl)ethanesulfonamide (Compound 19d). Compound 19d was obtained as a colorless sticky material in a yield of 87%; ¹H NMR (CDCl₃, 500 MHz): *δ* 8.29 (s, 1H), 7.60-7.59 (m, 4H), 7.39-7.34 (m, 2H), 7.32-7.30 (m, 4H), 7.28-7.27 (m, 2H), 7.25-7.24 (m, 2H), 7.20 (s, 1H), 6.66 (brs, 1H), 6.30 (d, *J* = 2.9 Hz, 1H), 5.37 (brs, 2H), 4.97 (dd, *J* = 5.6, 3.0 Hz, 1H), 4.82 (dd, *J* = 5.6, 2.7 Hz, 1H), 3.90-3.82 (m, 2H), 3.80-3.78 (m, 1H), 3.15 (q, *J* = 14.7, 7.3 Hz, 2H), 1.60 (s, 3H), 1.39 (t, *J* = 7.3 Hz, 3H), 1.29 (s, 3H), 1.02 (s, 9H); HRMS (ESI-Q-TOF) *m*/*z* [M + H]⁺ for C₃₈H₄₆N₅O₅S₂Si calculated 744.2704, found 744.2698.

### Preparation Example 1-6: Synthesis of Compounds 2a to 2d

Compounds 2a to 2d were synthesized in a yield of 78%, 81%, 80%, and 80%, respectively, using the known synthetic method (B).

### Preparation Example 2: Synthesis of Compounds 3 and 4a to 4d

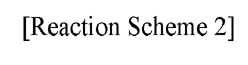

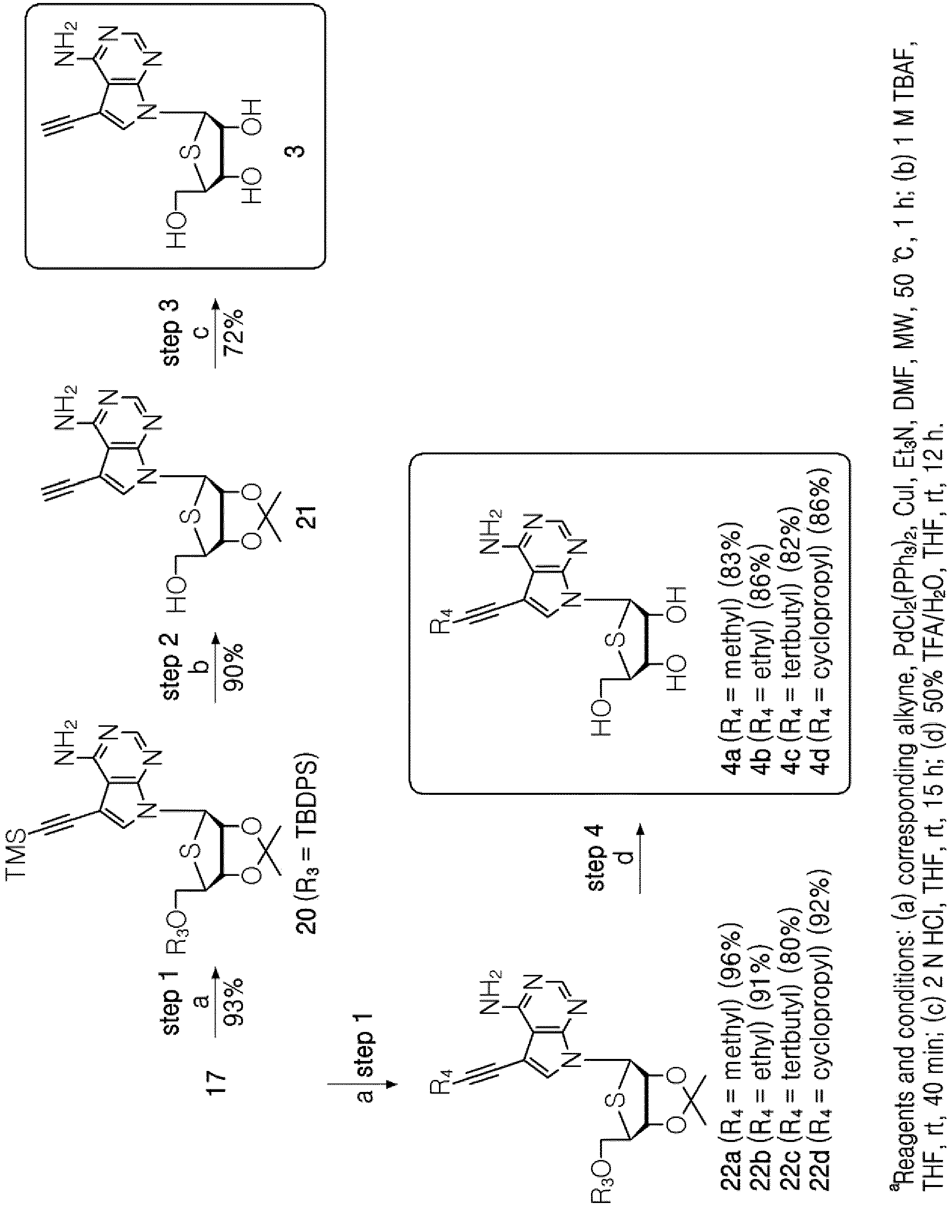

### Preparation Example 2-1: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-5-((trimethylsilyl)ethynyl)-7H-pyrrolo[2,3-d]pyrimidine-4-amine (Compound 20)

Known synthetic method (D): After cuprous iodide (25 mol%) and PdCl₂(PPh₃)₂ (10 mol%) were added to Compound 17, dimethylformamide-triethylamine (4:1) was added thereto, and the resulting mixture was substituted with nitrogen for 5 minutes. The corresponding alkyne was added thereto, and the resulting mixture was stirred at 50°C in a microwave for 1 hour. Extraction was performed with ethyl acetate and distilled water, the organic layer was partitioned, and then dried over anhydrous MgSO₄, and the remaining solid was removed by performing filtration under reduced pressure. The filtrate was concentrated under reduced pressure, and subjected to silica gel chromatography to obtain the compound.

Compound 20 was obtained as a sticky material in a yield of 93%; ¹H NMR (CDCl₃, 500 MHz): *δ* 8.24 (s, 1H), 7.65-7.62 (m, 4H), 7.42-7.39 (m, 3H), 7.37-7.34 (m, 4H), 6.19 (d, *J* = 2.6 Hz, 1H), 5.73 (brs, 2H), 4.89 (dd, *J* = 5.6, 2.6 Hz, 1H), 4.78 (dd, *J* = 5.6, 2.3 Hz, 1H), 3.87-3.84 (m, 1H), 3.79-3.73 (m, 2H), 1.57 (s, 3H), 1.27 (s, 3H), 1.07 (s, 9H), 0.24 (s, 9H); HRMS (ESI-Q-TOF) *m*/*z* [M + H]⁺ for C₃₅H₄₅N₄O₃SSi₂ calculated 657.2745, found 657.2739.

### Preparation Example 2-2: Synthesis of ((3aS,4R,6R,6aR)-6-(4-amino-5-ethynyl-7H-pyrrolo[2,3-d]pyrimidin-7-yl)-2,2-dimethyltetrahydrothieno[3,4-d][1,3]dioxol-4-yl)methanol (Compound 21)

Known synthetic method (E): After Compound 20 (0.58 g, 0.88 mmol) was dissolved in anhydrous tetrahydrofuran (5.8 mL), 1 M tetra-n-butylammonium fluoride (2.64 mL, 2.64 mmol) was added thereto. After the reaction solution was stirred at room temperature for 40 minutes, the reaction was terminated with ammonium chloride. After extraction was performed with ethyl acetate, the organic solvent layer was partitioned, washed with distilled water and brine, and then dried over anhydrous MgSO₄, and the remaining solid was removed by performing filtration under reduced pressure. The residue was concentrated under reduced pressure, and then subjected to silica gel chromatography to obtain Compound 21 (0.27 g, 90%).

¹H NMR (CD₃OD, 500 MHz): *δ* 8.12 (s, 1H), 7.78 (s, 1H), 6.27 (d, *J* = 3.0 Hz, 1H), 5.14 (dd, *J* = 5.3, 3.1 Hz, 1H), 4.97 (dd, *J* = 5.4, 2.2 Hz, 1H), 3.79-3.74 (m, 2H), 3.73 (s, 1H), 3.71-3.68 (m, 1H), 1.58 (s, 3H), 1.32 (s, 3H); HRMS (ESI-Q-TOF) *m*/*z* [M + H]⁺ for C₁₆H₁₉N₄O₃S calculated 347.1172, found 347.1168.

### Preparation Example 2-3: Synthesis of (2R,3R,4S,5R)-2-(4-amino-5-ethynyl-7H-pyrrolo[2,3-d]pyrimidin-7-yl)-5-(hydroxymethyl)tetrahydrothiophene-3,4-diol (Compound 3)

Known synthetic method (F): After Compound 21 (0.21 g, 0.60 mmol) was dissolved in anhydrous tetrahydrofuran, a 2 N aqueous hydrochloric acid solution (6 mL) was added thereto at low temperature, and the resulting mixture was stirred at room temperature for 15 hours. After the reaction was terminated, the resulting product was neutralized under stirring for 1 hour using a weakly basic cation exchange resin (Dowex^{®} 66 free base). After the remaining solid was removed by performing filtration under reduced pressure, the residue was concentrated under reduced pressure and subjected to silica gel chromatography to obtain Compound 3 (0.13 g, 72%) as a white solid.

### Preparation Example 2-4: Synthesis of Compounds 22a to 22d

Compounds 22a to 22d were synthesized by the known synthetic method (D).

7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-5-(prop-1-yn-1-yl)-7*H*-pyrrolo[2,3-*d*]pyrimidine-4-amine (Compound 22a). Compound 22a was obtained as a sticky material in a yield of 96%; ¹H NMR (CDCl₃, 400 MHz): *δ* 8.22 (s, 1H), 7.66-7.63 (m, 4H), 7.41-7.40 (m, 2H), 7.38-7.34 (m, 4H), 7.30 (s, 1H), 6.20 (d, *J* = 2.8 Hz, 1H), 5.74 (brs, 2H), 4.90 (dd, *J* = 5.6, 2.8 Hz, 1H), 4.80 (dd, *J* = 6.0, 5.8 Hz, 1H), 3.88 (dd, *J* = 10, 6.8 Hz, 1H), 3.82-3.75 (m, 2H), 2.07 (s, 3H), 1.58 (s, 3H), 1.28 (s, 3H), 1.08 (s, 9H); HRMS (ESI-Q-TOF) *m*/*z* [M + H]⁺ for C₃₃H₃₉N₄O₃SSi calculated 599.2507, found 599.2512.

5-(but-1-yn-1-yl)-7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-7*H*-pyrrolo[2,3-*d*]pyrimidine-4-amine (Compound 22b). Compound 22b was obtained as a sticky material in a yield of 91%; ¹H NMR (CDCl₃, 500 MHz): *δ* 8.22 (s, 1H), 7.65-7.62 (m, 4H), 7.42-7.39 (m, 2H), 7.37-7.34 (m, 4H), 7.30 (s, 1H), 6.19 (d, *J* = 2.7 Hz, 1H), 5.68 (brs, 2H), 4.89 (dd, *J* = 5.7, 2.8 Hz, 1H), 4.79 (dd, *J* = 5.6, 2.8 Hz, 1H), 3.87-3.85 (m, 1H), 3.80-3.77 (m, 1H), 3.76-3.74 (m, 1H), 2.43 (q, *J* = 14.9, 7.5 Hz, 2H), 1.57 (s, 3H), 1.27 (s, 3H), 1.23 (t, *J* = 7.5 Hz, 3 H), 1.06 (s, 9H); HRMS (ESI-Q-TOF) *m*/*z* [M + H]⁺ for C₃₄H₄₁N₄O₃SSi calculated 613.2663, found 613.2669.

7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-5-(3,3-dimethylbut-1-yn-1-yl)-7*H*-pyrrolo[2,3-d]pyrimidine-4-amine (Compound 22c). Compound 22c was obtained as a sticky material in a yield of 80%; ¹H NMR (CDCl₃, 500 MHz): *δ* 8.21 (s, 1H), 7.64 (t, *J* = 6.4 Hz, 4H), 7.42-7.40 (m, 2H), 7.38-7.34 (m, 4H), 7.32 (s, 1H), 6.20 (d, *J* = 2.6 Hz, 1H), 5.83 (brs, 2H), 4.88 (dd, *J* = 5.6, 2.7 Hz, 1H), 4.77 (dd, *J* = 5.5, 2.5 Hz, 1H), 3.87 (dd, *J* = 9.7, 6.2 Hz, 1H), 3.79-3.73 (m, 2H), 1.57 (s, 3H), 1.31 (s, 9H), 1.26 (s, 3H), 1.07 (s, 9H); HRMS (ESI-Q-TOF) *m*/*z* [M + H]⁺ for C₃₆H₄₅N₄O₃SSi calculated 641.2976, found 641.2991.

7-((3a*R*,4*R*,6*R*,6a*S*)-6-(((*tert*-butyldiphenylsilyl)oxy)methyl)-2,2-dimethyltetrahydrothieno[3,4-*d*][1,3]dioxol-4-yl)-5-(cyclopropylethynyl)-7*H*-pyrrolo[2,3-d]pyrimidine-4-amine (Compound 22d). Compound 22d was obtained as a sticky material in a yield of 92%; ¹H NMR (CDCl₃, 400 MHz): *δ* 8.21 (s, 1H), 7.66-7.63 (m, 4H), 7.44-7.40 (m, 2H), 7.39-7.34 (m, 4H), 7.31 (s, 1H), 6.19 (d, *J* = 3.2 Hz, 1H), 5.93 (brs, 2H), 4.88 (dd, *J* = 5.9, 3.2 Hz, 1H), 4.79 (dd, *J* = 5.9, 2.7 Hz, 1H), 3.89-3.85 (m, 1H), 3.82-3.73 (m, 2H), 1.58 (s, 3H), 1.49-1.44 (m, 1H), 1.27 (s, 3H), 1.07 (s, 9H), 0.92-0.85 (m, 2H), 0.79-0.75 (m, 2H); HRMS (ESI-Q-TOF) *m*/*z* [M + H]⁺ for C₃₅H₄₁N₄O₃SSi calculated 625.2663, found 625.2667.

### Preparation Example 2-5: Synthesis of Compounds 4a to 4d

Compounds 4a to 4d were synthesized in a yield of 83%, 86%, 82%, and 86%, respectively, by the known synthetic method (B).

### Preparation Example 3: Synthesis of Compounds 5 and 6

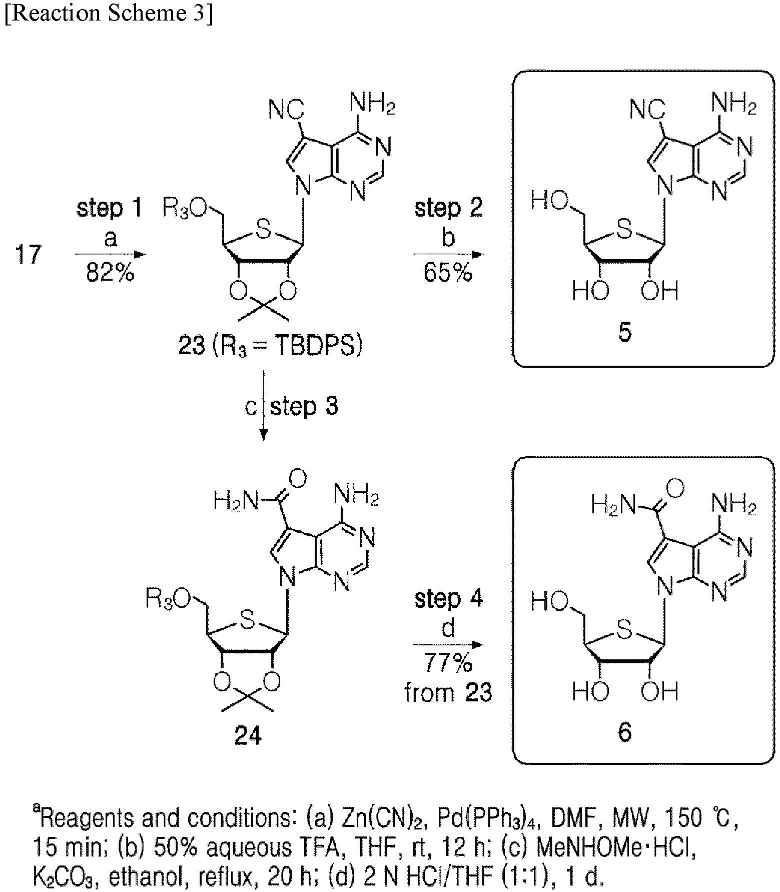

### Preparation Example 3-1: Synthesis of 4-amino-7-((3aR,4R,6R,6aS)-6-(((tert-bulyldiphenylsilyl)oxy)methyl)-2,2-dimethyltetrahydrothieno[3,4-d][1,3]dioxol-4-yl)-7H-pyrrolo[2,3-d]pyrimidine-5-carbonitrile (Compound 23)

After Compound 17 (0.42 g, 0.62 mmol) was dissolved in dimethylformamide (5 mL), zinc cyanide (0.10 g, 0.92 mmol) and Pd(PPh₃)₄ (0.071 g, 0.06 mmol) were added thereto, and the resulting mixture was stirred at 150°C in a microwave for 15 minutes. After the reaction is completed, the remaining solid was removed by performing filtration under reduced pressure at room temperature, and then the residue was concentrated under reduced pressure. The residue was subjected to silica gel chromatography to obtain Compound 23 (0.29 g, 82%).

¹H NMR (CDCl₃, 400 MHz): *δ* 8.36 (s, 1H), 7.78 (s, 1H), 7.66-7.62 (m, 4H), 7.45-7.35 (m, 6H), 6.17 (d, *J* = 2.4 Hz, 1H), 4.91 (dd, *J*₁ = 2.4 Hz, *J*₂ = 5.6 Hz, 1H), 4.82 (d, *J* = 5.6 Hz, 1H), 3.81 (s, 3H), 1.61 (s, 3H), 1.30 (s, 3H), 1.09 (s, 9H). HRMS (ESI-Q-TOF) *m*/*z* [M + H]⁺ for C₃₁H₃₆N₅O₃SSi calculated 586.2303, found 586.2311.

### Preparation Example 3-2: Synthesis of 4-amino-7-((2R,3R,4S,5R)-3,4-dihydroxy-5-(hydroxymethyl)tetrahydrothiophen-2-yl)-7H-pyrrolo[2,3-d]pyrimidine-5-carbonitrile (Compound 5)

After Compound 23 (0.23 g, 0.42 mmol) was dissolved in tetrahydrofuran (5 mL), an aqueous solution of 50% trifluoroacetic acid (5 mL) was added thereto at 0°C. After the resulting mixture was stirred at room temperature for 12 hours, the mixture was evaporated together with toluene when the reaction was completed. Purification was performed by silica gel column chromatography to obtain Compound 5 (0.078 g, 65%).

### Preparation Example 3-3: Synthesis of 4-amino-7-((3aR,4R,6R,6aS)-6-(((tert-bulyldiphenylsilyl)oxy)methyl)-2,2-dimethyltetrahydrothieno[3,4-d][1,3]dioxol-4-yl-7H-pyrrolo[2,3-d]pyrimidine-5-carboxamide (Compound 24)

After Compound 23 (0.25 g, 0.77 mmol) was dissolved in ethanol-distilled water, dimethylhydroxylamine hydrochloride (0.18 g, 1.93 mmol) and potassium carbonate (0.32 g, 2.32 mmol) were added thereto, and the resulting mixture was stirred under reflux for 20 hours. The resulting solid was filtered under reduced pressure, washed with methanol, and then dried. Compound 24 was used in the next reaction without any purification process after synthesis.

### Preparation Example 3-4: Synthesis of 4-amino-7-((2R,3R,4S,5R)-3,4-dihydroxy-5-(hydroxymethyl)tetrahydrothiophen-2-yl)-7H-pyrrolo[2,3-d]pyrimidine-5-carboxamide (Compound 6)

Compound 6 (105 mg, 77% in 2 steps) was synthesized from Compound 24 using the known synthetic method (F).

The following Tables 1 and 2 summarize the ¹H NMR and high resolution mass spectrometry (HRMS) data of compounds represented by the following Chemical Formula I, respectively.

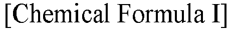

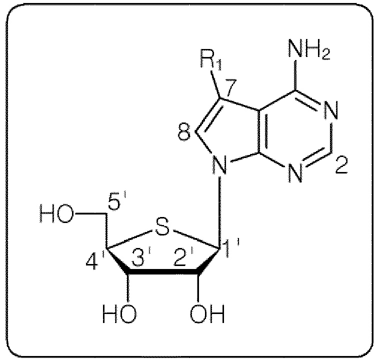

**[Table 1]**

| Compound | NMR Solvent/ Frequen cy | ¹H NMR data (Chemical shift, *δ* ppm) | | | | | | | | |
|---|---|---|---|---|---|---|---|---|---|---|
| | | 1' | 2' | 3' | 4' | 5' | 2 | 7 (R₁ group) | | 8 |
| 1a (R₁ = 2-furanyl) | DMSO-*d₆*/ 500 MHz | 6.17 (d, *J* = 7.0 Hz, 1H) | 4.48-4.45 (m, 1H) | 4.19-4.18 (m, 1H) | 3.29-3.28 (m, 1H) | 3.78-3.75 (m, 1H), 3.63-3.58 (m, 1H) | 8.13 (s, 1H) | 7.95 (s, 1H), 6.72 (d, *J* = 3.0 Hz, 1H), 6.62-6.61 (m, 1H) | 7.78 (s, 1H) | |
| 1b(R₁ = 2-thiofuranyl) | CD₃OD /500 MHz | 6.22 (d, *J* = 5.9 Hz, 1H) | 4.52 (q, *J* = 5.7, 3.7 Hz, 1H) | 4.29 (t, *J* = 3.8 Hz, 1H) | 3.48 (q, *J* = 9.4, 4.8 Hz, 1H) | 3.88-3.80 (m, 2H) | 8.13 (s, 1H) | 7.42 (d, *J* = 5.0 Hz, 1H), 7.14-7.11 (m, 2H) | 7.66 (s, 1H) | |
| 2a(R₁ = vinyl) | DMSO-*d₆*/500 MHz | 6.12 (d, *J* = 7.0 Hz, 1H) | 4.46-4.41 (m, 1H) | 4.18-4.14 (m, 1H) | 3.24 (m, 1H) | 3.77-3.72 (m, 1H), 3.61-3.55 (m, 1H) | 8.04 (s, 1H) | 7.10 (dd, *J* = 17.2, 10.9 Hz, 1H), 5.59 (d, *J* = 17.1 Hz, 1H), 5.11 (d, *J* = 11.0 Hz, 1H), | 7.77 (s, 1H) 3.27- | |
| 2b (R₁ =phenyl) | DMSO-*d*₆/500 MHz | 6.18 (d, *J* = 6.9 Hz, 1H) | 4.51-4.47 (m, 1H) | 4.19-4.16 (m, 1H) | 3.29-3.25 (m, 1H) | 3.77-3.71 (m, 1H), 3.61-3.56 (m, 1H) | 8.14 (s, 1H) | 7.50-7.46 (m, 4H), 7.38-7.34 (m, 1H) | 7.66 (s, 1H) | |
| 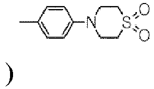 2c(R₁ = | DMSO-*d*₆/500 MHz | 6.18 (d, *J* = 6.9 Hz, 1H) | 4.49-4.47 (m, 1H) | 4.18-4.17 (m, 1H) | 3.29-3.27 (m, 1H) | 3.75-3.72 (m, 1H), 3.60-3.57 (m, 1H) | 8.13 (s, 1H) | 7.35 (d, *J* = 8.4 Hz, 2H), 7.13 (d, *J* = 8.5 Hz, 2H), 3.84 (s, 4H), 3.14 (s, 4H) | 7.54 (s, 1H) | |
| 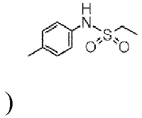 2d(R₁ = | DMSO-*d*₆/500 MHz) | 6.18 (d, *J* = 6.9 Hz, 1H) | 4.49-4.46 (m, 1H) | 4.19-4.17 (m, 1H) | 3.29-3.27 (m, 1H) | 3.76-3.72 (m, 1H), 3.61-3.57 (m, 1H) | 8.14 (s, 1H) | 9.87 (s, 1H), 7.42 (merged dd, *J*₁ = *J*₂ = 8.2 Hz, 2H), 7.31 (merged dd, *J*₁ = *J*₂ = 8.1 Hz, 2H), 3.13 (q, *J* = 14.4, 7.1 Hz, 2H), 1.22 (t, *J* = 7.2 Hz, 3H) | 7.60 (s, 1H) | |
| 3(R₁ = acetylene) | DMSO-*d*₆/500 MHz | 6.06 (d, *J* = 6.9 Hz, 1H) | 4.45-4.42 (m, 1H) | 4.16-4.14 (m, 1H) | 3.28-3.25 (m, 1H) | 3.76-3.71 (m, 1H), 3.61-3.57 (m, 1H) | 8.12 (s, 1H) | 4.28 (s, 1H) | 7.93 (s, 1H) | |
| 4a(R₁ =prop-1-yn-1-yl) | DMSO-*d₆*/500 MHz | 6.05 (d, *J* = 6.7 Hz, 1H) | 4.42-4.39 (m, 1H) | 4.16-4.13 (m, 1H) | 3.27-3.24 (m, 1H) | 3.75-3.70 (m, 1H), 3.61-3.56 (m, 1H) | 8.10 (s, 1H) | 2.08 (s, 3H) | 7.75 (s, 1H) | |
| 4b(R₁ = but-1-yn-1-yl) | DMSO-*d*₆/500 MHz) | 6.06 (d, *J* = 6.8 Hz, 1H) | 4.43-4.40 (m, 1H) | 4.16-4.13 (m, 1H) | 3.27-3.24 (m, 1H) | 3.75-3.70 (m, 1H), 3.60-3.56 (m, 1H) | 8.10 (s, 1H) | 2.47 (q, *J* = 14.9, 7.4 Hz, 2H), 1.17 (t, *J* = 7.4 Hz, 3H) | 7.76 (s, 1H) | |
| 4c(R₁ = 3,3-dimethylbut-1-yn-1-yl) | DMSO-*d*₆/500 MHz | 6.06 (d, *J* = 7.0 Hz, 1H) | 4.45-4.41 (m, 1H) | 4.16-4.13 (m, 1H) | 3.27-3.24 (m, 1H) | 3.76-3.71 (m, 1H), 3.61-3.55 (m, 1H) | 8.11 (s, 1H) | 1.31 (s, 9H) | 7.76 (s, 1H) | |
| 4d(R₁ = cyclopropyl ethynyl) | CD₃OD /500 MHz | 6.12 (d, *J* = 5.9 Hz, 1H) | 4.44 (dd, *J* = 5.8, 3.7 Hz, 1H) | 4.25-4.23 (m, 1H) | 3.48-3.44 (m, 1H) | 3.88-3.79 (m, 2H) | 8.10 (s, 1H) | 1.56-1.50 (m, 1H), 0.93-0.88 (m, 2H), 0.78-0.74 (m, 2H) | 7.68 (s, 1H) | |
| 5(R₁ = CN) | CD₃OD /400 MHz | 6.14 (d, *J* = 5.6 Hz, 1H) | 4.47 (dd, *J*₁ = 4.0 Hz, *J*₂ = 5.6 Hz, 1H) | 4.23 (supe rimpo sed dd, *J*₁ = *J*₂ = 4.0 Hz, 1H) | 3.51-3.46 (m, 1H) | 3.87-3.83 (m, 2H) | 8.40 (s, 1H) | - | 8.20 (s, 1H) | |
| 6(R₁ = CONH₂) | CD₃OD /400 MHz | 6.22 (d, *J* = 6.0 Hz, 1H) | 4.49 (dd, *J*₁ = 4.0 Hz, *J*₂ = 6.0 Hz, 1H) | 4.29 (supe rimpo sed dd, *J*₁ = *J*₂ = 4.0 Hz, 1H) | 3.58-3.53 (m, 1H) | 3.95-3.89 (m, 2H) | 8.35 (s, 1H) | - | 8.13 (s, 1H) | |

**[Table 2]**

| Compound | HRMS, *m*/*z* [M + H]⁺ | Molecular formula |
|---|---|---|
| 1a (R₁ = 2-furanyl) | 349.0974 | C₁₅H₁₇N₄O₄S |
| 1b (R₁ = 2-thiofuranyl) | 365.0749 | C₁₅H₁₇N₄O₃S₂ |
| 2a(R₁ = vinyl) | 309.1018 | C₁₃H₁₇N₄O₃S |
| 2b (R₁ = phenyl) | 359.1177 | C₁₇H₁₉N₄O₃S |
| 2c(R₁ 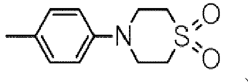 ) | 492.1388 | C₂₁H₂₆N₅O₅S₂ |
| 2d(R₁ = 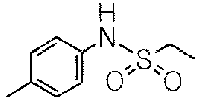 ) | 466.1225 | C₁₉H₂₄N₅O₅S₂ |
| 3 (R₁ = acetylene) | 307.0863 | C₁₃H₁₅N₄O₃S |
| 4a(R₁ = prop-1-yn-1-yl) | 321.1013 | C₁₄H₁₇N₄O₃S |
| 4b(R₁ = but-1-yn-1-yl) | 335.1157 | C₁₅H₁₉N₄O₃S |
| 4c(R₁ = 3,3-dimethylbut-1-yn-1-yl) | 363.1475 | C₁₇H₂₃N₄O₃S |
| 4d(R₁ = cyclopropylethynyl) | 347.1159 | C₁₆H₁₉N₄O₃S |
| 5(R₁ = CN) | 307.0808 | C₁₂H₁₄N₅O₃S |
| 6(R₁ = CONH₂) | 326.0921 | C₁₂H₁₆N₅O₄S |

### Preparation Example 4: Synthesis of Compounds 7a to 7h

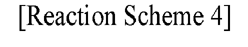

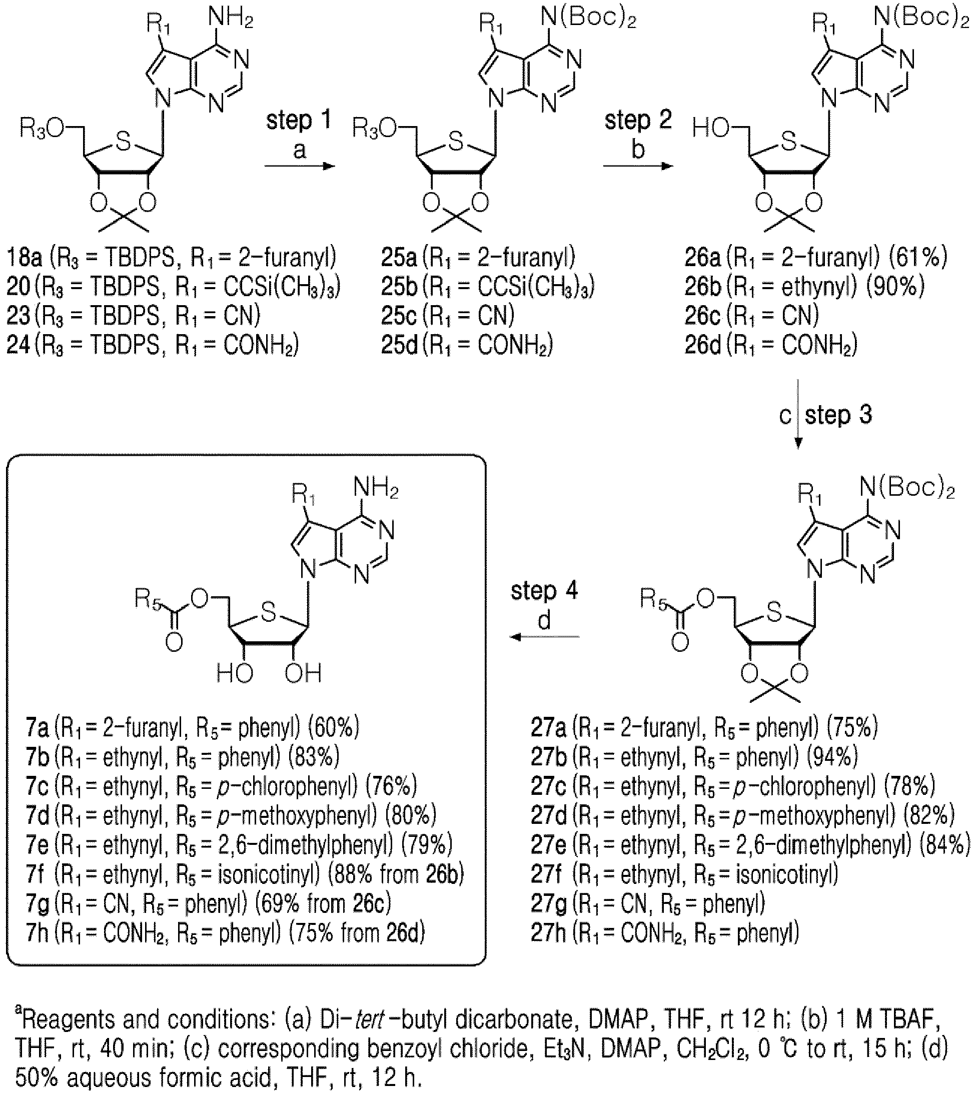

### Preparation Example 4-1: Synthesis of Compounds 25a to 25d

After Compounds 18a, 20, 23, and 24 (1 eq.) were each dissolved in anhydrous tetrahydrofuran, 4-dimethylaminopyridine (3 eq.) and di-tert-butyl dicarbonate (6 eq.) were added thereto. After the reaction solution was stirred at room temperature for 12 hours, it was confirmed that the reaction was terminated. After concentration under reduced pressure, extraction was performed with distilled water and ethyl acetate, and the organic solvent layer was partitioned and then dried over anhydrous MgSO₄. After the remaining solid was removed by performing filtration under reduced pressure, the residue was concentrated under reduced pressure to obtain crude Compounds 25a to 25d, respectively.

### Preparation Example 4-2: Synthesis of Compounds 26a to 26d

Compounds 26a to 26d were synthesized using the known synthetic method (E).

*Tert*-butyl (*tert*-butoxycarbonyl)(5-(furan-2-yl)-7-((3a*R*,4*R*,6*R*,6a*S*)-6-(hydroxymethyl)-2,2-dimethyltetrahydrothieno[3,4-*d*][1,3]dioxol-4-yl)-7*H*-pyrrolo[2,3-*d*]pyrimidin-4-yl)carbamate (Compound 26a). Compound 26a was obtained as a sticky material in a yield of 61%; ¹H NMR (CDCl₃, 400 MHz): *δ* 8.84 (s, 1H), 7.67 (s, 1H), 7.42 (d, *J* = 1.8 Hz, 1H), 6.46-6.43 (m, 2H), 6.23 (d, *J* = 3.6 Hz, 1H), 5.28 (dd, *J* = 5.5, 3.6 Hz, 1H), 4.98 (dd, *J* = 5.5, 1.3 Hz, 1H), 4.01 (s, 2H), 3.89 (s, 2H), 1.65 (s, 3H), 1.34 (s, 3H), 1.27 (s, 18H); HRMS (ESI-Q-TOF) *m*/*z* [M + H]⁺ for C₂₈H₃₇N₄O₈S calculated 589.2327, found 589.2322.

*Tert*-butyl (*tert*-butoxycarbonyl)(5-furan-7-((3a*R*,4*R*,6*R*,6a*S*)-6-(hydroxymethyl)-2,2-dimethyltetrahydrothieno[3,4-*d*][1,3]dioxol-4-yl)-7*H*-pyrrolo[2,3-*d*]pyrimidin-4-yl)carbamate (Compound 26b). Compound 26b was obtained as a sticky material in a yield of 90%; ¹H NMR (CD₃OD, 400 MHz): *δ* 8.78 (s, 1H), 8.30 (s, 1H), 6.46 (d, *J* = 2.8 Hz, 1H), 5.21 (dd, *J* = 5.6, 3.2 Hz, 1H), 5.02 (dd, *J* = 5.2, 1.2 Hz, 1H), 3.79-3.74 (m, 3H), 3.67 (s, 1H), 1.61 (s, 3H), 1.34 (s, 21H); HRMS (ESI-Q-TOF) *m*/*z* [M + H]⁺ for C₂₆H₃₅N₄O₇S calculated 547.2221, found 547.2214.

Compounds 26c and 26d were used in the next reaction without any purification process.

### Preparation Example 4-3: Synthesis of Compounds 27a to 27h

Known synthetic method (G): After Compounds 26a to 26d (1 eq.) were dissolved in dichloromethane, 4-dimethylaminopyridine (0.08 eq.), triethylamine (2.5 eq.), and benzoyl chloride (1.1 eq.) were each added thereto at 0°C. After the resulting mixture was stirred at room temperature for 15 hours, it was confirmed that the reaction was terminated. The reaction solution was extracted with distilled water and ethyl acetate, and the organic solvent layer was partitioned, and then dried over anhydrous MgSO₄. After the remaining solid was removed by performing filtration under reduced pressure, the residue was concentrated under reduced pressure. Purification was performed by silica gel column chromatography to obtain the compound.

((3a*S*,4*R*,6*R*,6a*R*)-6-(4-(bis(*tert*-butoxycarbonyl)amino)-5-(furan-2-yl)-7*H*-pyrrolo[2,3-*d*]pyrimidin-7-yl)-2,2-dimethyltetrahydrothieno[3,4-*d*][1,3]dioxol-4-yl)methyl benzoate (Compound 27a). Compound 27a was synthesized from Compound 26a and obtained as a sticky material in a yield of 75%; ¹H NMR (CDCl₃, 400 MHz): *δ* 8.87 (s, 1H), 8.03 (dd, *J* = 8.6, 1.3 Hz, 2H), 7.74 (s, 1H), 7.58-7.54 (m, 1H), 7.44-7.41 (m, 2H), 7.39-7.38 (m, 1H), 6.44 (d, *J* = 2.7 Hz, 1H), 6.42-6.40 (m, 2H), 5.25 (dd, *J* = 5.4, 2.2 Hz, 1H), 5.04 (dd, *J* = 5.4, 3.2 Hz, 1H), 4.70 (dd, *J* = 11.4, 7.3 Hz, 1H), 4.50 (dd, *J* = 11.4, 6.4 Hz, 1H), 4.02 (td, *J* = 6.8, 3.2 Hz, 1H), 1.64 (s, 3H), 1.34 (s, 3H), 1.29 (s, 9H), 1.26 (s, 9H); HRMS (ESI-Q-TOF) *m*/*z* [M + H]⁺ for C₃₅H₄₁N₄O₉S calculated 693.2589, found 693.2585.

((3a*S*,4*R*,6*R*,6a*R*)-6-(4-(bis(*tert*-butoxycarbonyl)amino)-5-ethynyl-7*H*-pyrrolo[2,3-*d*]pyrimidin-7-yl)-2,2-dimethyltetrahydrothieno[3,4-*d*][1,3]dioxol-4-yl)methyl benzoate (Compound 27b). Compound 27b was synthesized from Compound 26b and obtained as a colorless sticky material in a yield of 94%; ¹H NMR (CDCl₃, 500 MHz): *δ* 8.86 (s, 1H), 8.04-8.02 (m, 2H), 7.70 (s, 1H), 7.58-7.55 (m, 1H), 7.46-7.43 (m, 2H), 6.34 (d, *J* = 2.4 Hz, 1H), 5.21 (dd, *J* = 5.6, 2.4 Hz, 1H), 5.04 (dd, *J* = 5.6, 3.3 Hz, 1H), 4.66 (dd, *J* = 11.4, 7.4 Hz, 1H), 4.51 (dd, *J* = 11.4, 6.4 Hz, 1H), 4.02-3.99 (m, 1H), 3.14 (s, 1H), 1.63 (s, 3H), 1.38 (s, 18H), 1.33 (s, 3H); HRMS (ESI-Q-TOF) *m*/*z* [M+H]⁺ for C₃₃H₃₉N₄O₈S calculated 651.2483, found 651.2485.

((3a*S*,4*R*,6*R*,6a*R*)-6-(4-(bis(*tert*-butoxycarbonyl)amino)-5-ethynyl-7*H*-pyrrolo[2,3-*d*]pyrimidin-7-yl)-2,2-dimethyltetrahydrothieno[3,4-*d*][1,3]dioxol-4-yl)methyl 4-chlorobenzoate (Compound 27c). Compound 27c was synthesized from Compound 26b and obtained as a sticky material in a yield of 78%; ¹H NMR (CD₃OD, 500 MHz): *δ* 8.80 (s, 1H), 8.04 (s, 1H), 7.99-7.96 (m, 2H), 7.49-7.48 (m, 2H), 6.44 (d, *J* = 2.2 Hz, 1H), 5.44 (dd, *J* = 5.5, 2.2 Hz, 1H), 5.26 (dd, *J* = 5.5, 2.7 Hz, 1H), 4.65 (dd, *J* = 11.3, 7.7 Hz, 1H), 4.45 (dd, *J* = 11.3, 6.6 Hz, 1H), 4.03-4.0 (m, 1H), 3.66 (s, 1H), 1.60 (s, 3H), 1.35 (s, 3H), 1.33 (s, 18H); HRMS (ESI-Q-TOF) *m*/*z* [M+H]⁺ for C₃₃H₃₈ClN₄O₈S calculated 685.2093, found 685.2092.

((3a*S*,4*R*,6*R*,6a*R*)-6-(4-(bis(*tert*-butoxycarbonyl)amino)-5-ethynyl-7*H*-pyrrolo[2,3-*d*]pyrimidin-7-yl)-2,2-dimethyltetrahydrothieno[3,4-*d*][1,3]dioxol-4-yl)methyl 4-methoxybenzoate (Compound 27d). Compound 27d was synthesized from Compound 26b and obtained as a sticky material in a yield of 82%; ¹H NMR (CDCl₃, 500 MHz): *δ* 8.85 (s, 1H), 7.98-7.97 (m, 2H), 7.71 (s, 1H), 6.92-6.90 (m, 2H), 6.35 (d, *J* = 2.3 Hz, 1H), 5.18 (dd, *J* = 5.5, 2.4 Hz, 1H), 5.02 (dd, *J* = 5.5, 3.2 Hz, 1H), 4.63 (dd, *J* = 11.4, 7.3 Hz, 1H), 4.47 (dd, *J* = 11.4, 6.4 Hz, 1H), 4.01-3.97 (m, 1H), 3.84 (s, 3H), 3.13 (s, 1H), 1.62 (s, 3H), 1.37 (s, 18H), 1.33 (s, 3H); HRMS (ESI-Q-TOF) *m*/*z* [M+H]⁺ for C₃₄H₄₁N₄O₉S calculated 681.2589, found 681.2591.

((3a*S*,4*R*,6*R*,6a*R*)-6-(4-(bis(*tert*-butoxycarbonyl)amino)-5-ethynyl-7*H*-pyrrolo[2,3-*d*]pyrimidin-7-yl)-2,2-dimethyltetrahydrothieno[3,4-*d*][1,3]dioxol-4-yl)methyl 4-dimethylbenzoate (Compound 27e). Compound 27e was synthesized from Compound 26b and obtained as a sticky material in a yield of 84%; ¹H NMR (CDCl₃, 400 MHz): *δ* 8.84 (s, 1H), 7.64 (s, 1H), 7.21-7.17 (m, 1H), 7.05-7.02 (m, 2H), 6.33 (d, *J* = 3.0 Hz, 1H), 5.18 (dd, *J* = 6.1, 3.0 Hz, 1H), 5.02 (dd, *J* = 5.5, 3.0 Hz, 1H), 4.64-4.53 (m, 2H), 3.98 (td, *J* = 6.7, 3.0 Hz, 1H), 3.15 (s, 1H), 2.32 (s, 6H), 1.62 (s, 3H), 1.38 (s, 18H), 1.31 (s, 3H); HRMS (ESI-Q-TOF) *m*/*z* [M+H]⁺ for C₃₅H₄₃N₄O₈S calculated 679.2796, found 679.2791.

((3a*S*,4*R*,6*R*,6a*R*)-6-(4-(bis(*tert*-butoxycarbonyl)amino)-5-ethynyl-7*H*-pyrrolo[2,3-*d*]pyrimidin-7-yl)-2,2-dimethyltetrahydrothieno[3,4-*d*][1,3]dioxol-4-yl)methyl isonicotinate (Compound 27f). Compound 27f was synthesized from Compound 26b, and used in the next reaction without any purification process.

Compounds 27g and 27h were synthesized from Compounds 26c and 26d, respectively, and used in the next reaction without any purification process.

### Preparation Example 4-4: Synthesis of Compounds 7a to 7h

Known synthetic method (H): A 50% aqueous formic acid solution and tetrahydrofuran were added to Compounds 27a to 27h, and the resulting mixture was stirred at room temperature for 12 hours. After it was confirmed that the reaction was terminated, the solution was neutralized under stirring for 1 hour using a basic cation exchange resin (Dowex^{®} 66 free base). After the remaining solid was removed by performing filtration under reduced pressure, the residue was concentrated under reduced pressure and purified by silica gel column chromatography to obtain Compounds 7a to 7h in a yield of 60%, 83%, 76%, 80%, 79%, 88% (2 steps), 69% (2 steps), and 75% (2 steps), respectively.

### Preparation Example 5: Synthesis of Compounds 8a to 8e

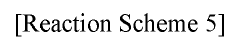

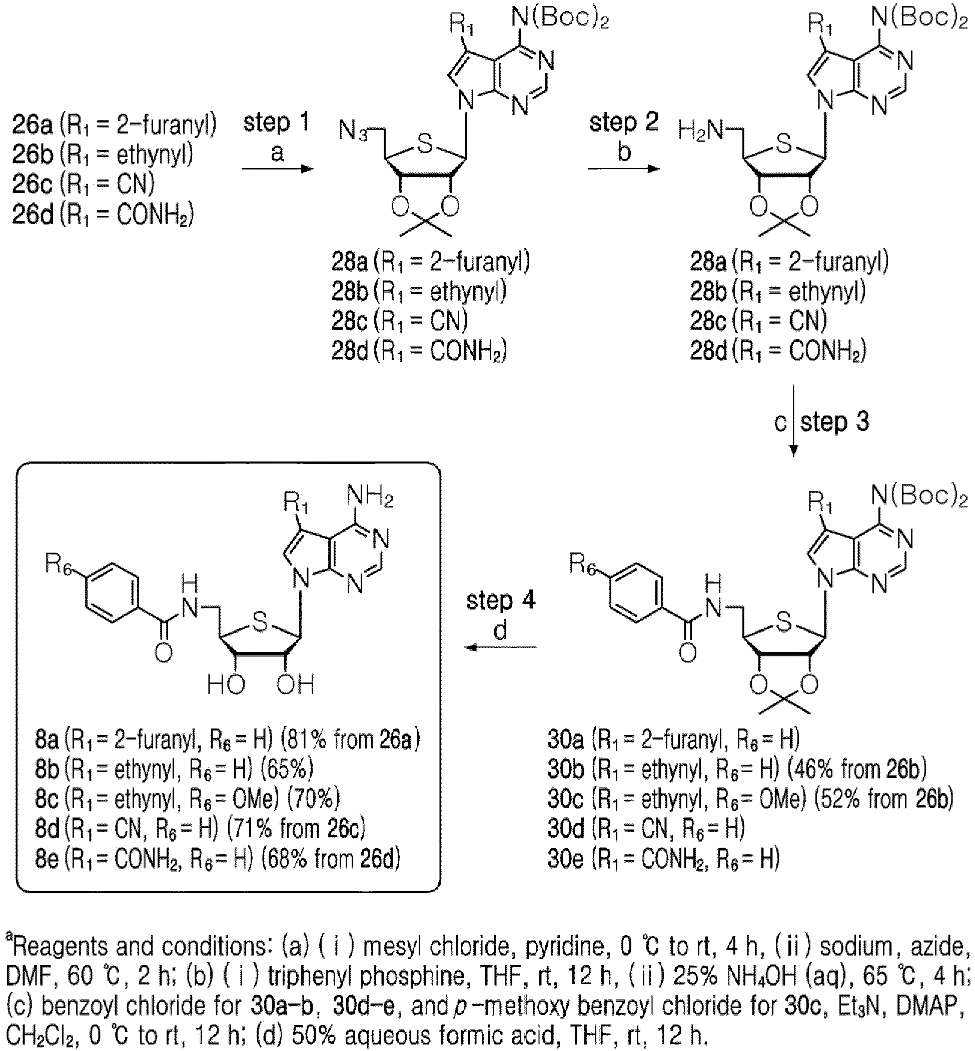

### Preparation Example 5-1: Synthesis of Compounds 28a to 28d

After Compounds 26a to 26d (1 eq.) were dissolved in pyridine (15 mL/mmol), methanesulfonyl chloride (2 eq.) was added dropwise thereto at 0°C under stirring. After stirring at room temperature for 4 hours, the reaction was terminated with a saturated sodium bicarbonate solution. Moreover, extraction was performed three times with ethyl acetate. The organic solvent layer was dried over anhydrous MgSO₄, and then filtered under reduced pressure, and the residue concentrated under reduced pressure was used in the next reaction without any purification process. After a crude methanesulfonyl compound was dissolved in dimethylformamide (5.5 mL/mmol), sodium azide (3 eq.) was added dropwise thereto under stirring. The reaction solution was heated at 60°C for 2 hours. Volatile materials were concentrated and evaporated under reduced pressure, and the residue was partitioned between ethyl acetate and water. The organic solvent layer was dried over magnesium sulfate, filtered under reduced pressure, and concentrated under reduced pressure to obtain crude Compounds 28a to 28d, respectively. Compounds 28a to 28d were immediately used in the next reaction without any purification process.

### Preparation Example 5-2: Synthesis of Compounds 29a to 29d

After triphenylphosphine (2 eq.) was added to Compounds 28a to 28d (1 eq.) dissolved in tetrahydrofuran (27 mL/mmol), the resulting mixture was stirred at room temperature for 12 hours. After 25% aqueous ammonia (1.8 mL/mmol) was added to the reaction solution, the resulting mixture was stirred at 65°C for 4 hours. Volatile materials were evaporated and crude Compounds 29a to 29d were immediately used in the next reaction without any purification process.

### Preparation Example 5-3: Synthesis of Compounds 30a to 30e

Compound 30a was synthesized from Compound 29a, Compounds 30b and 30c were synthesized from Compound 29b, and Compounds 30d and 30e were synthesized from Compounds 29d and 29e, respectively, using the general synthetic method (G) (Compounds 30a, 30b, 30d, and 30e were from benzoyl chloride, and Compound 30c was from 4-methoxybenzoyl chloride). Crude Compounds 30a, 30d, and 30e were used in the reaction without any purification process, whereas Compounds 30b and 30c were purified by silica gel column chromatography.

Tert-butyl (7-((3a*R*,4*R*,6*R*,6a*S*)-6-(benzamidomethyl)-2,2-dimethyltetrahydrothieno[3,4-*d*][1,3]dioxol-4-yl)-5-ethynyl-7*H*-pyrrolo[2,3-*d*]pyrimidin-4-yl)(tert-butoxycarbonyl)carbamate (Compound 30b). Compound 30b was synthesized from Compound 26b in 3 steps and obtained as a sticky material in a yield of 46%: ¹H NMR (CD₃OD, 500 MHz): *δ* 8.81 (s, 1H), 8.14 (s, 1H), 7.82 (d, *J* = 7.3 Hz, 2H), 7.54 (merged dd, *J*₁ = *J*₂ = 7.3 Hz, 1H), 7.46 (merged dd, *J*₁ = *J*₂ = 7.8 Hz, 2H), 6.44 (d, *J* = 2.6 Hz, 1H), 5.36 (dd, *J* = 5.6, 2.7 Hz, 1H), 5.12 (dd, *J* = 5.6, 2.9 Hz, 1H), 3.93-3.91 (m, 1H), 3.86 (dd, *J* = 13.5, 8.3 Hz, 1H), 3.68 (s, 1H), 3.62 (dd, *J* = 13.5, 6.6 Hz, 1H), 1.59 (s, 3H), 1.34 (s, 18H), 1.33 (s, 3H); HRMS (ESI-Q-TOF) *m*/*z* [M + H]⁺ for C₃₃H₄₀N₅O₇S calculated 650.2643, found 650.2634.

Tert-butyl(tert-butoxycarbonyl)(5-ethynyl-7-((3a*R*,4*R*,6*R*,6a*S*)-6-((4-methoxybenzamido)methyl)-2,2-dimethyltetrahydrothieno[3,4-*d*][1,3]dioxol-4-yl)-7*H-*pyrrolo[2,3-d]pyrimidin-4-yl)carbamate (Compound 30c). Compound 30c was synthesized from Compound 26b in 3 steps and obtained as a sticky material in a yield of 52%: ¹H NMR (CDCl₃, 400 MHz): *δ* 8.77 (s, 1H), 7.73 (s, 1H), 7.71 (d, *J* = 3.6 Hz, 2H), 6.90 (d, *J* = 9.1 Hz, 2H), 6.62 (brs, 1H), 6.29 (d, *J* = 3.0 Hz, 1H), 5.20 (dd, *J* = 5.5, 2.4 Hz, 1H), 4.98 (dd, *J* = 5.5, 3.0 Hz, 1H), 3.89-3.84 (m, 2H), 3.82 (s, 3H), 3.80-3.75 (m, 1H), 3.16 (s, 1H), 1.59 (s, 3H), 1.38 (s, 18H), 1.30 (s, 3H); HRMS (ESI-Q-TOF) *m*/*z* [M + H]⁺ for C₃₄H₄₂N₅O₈S calculated 680.2749, found 680.2739.

### Preparation Example 5-4: Synthesis of Compounds 8a to 8e

Compounds 8a to 8e were synthesized from Compounds 30a to 30e in a yield of 81% (4 steps), 65%, 70%, 71% (4 steps), and 68% (4 steps), respectively, by the known synthetic method (H).

The following Tables 3 and 4 summarize the ¹H NMR and high resolution mass spectrometry (HRMS) data of compounds represented by the following Chemical Formula II, respectively.

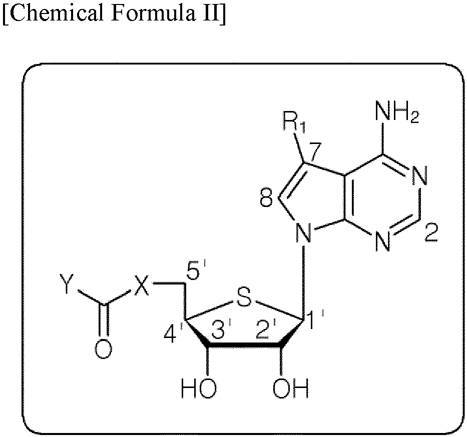

**[Table 3]**

| Comp ound | NMR Solve nt/Fre quenc y | | ¹H NMR data (Chemical shift, *δ* ppm) | | | | | | | |
|---|---|---|---|---|---|---|---|---|---|---|
| | | 1' | 2' | 3' | 4' | 5' | Y group | 2 | 7 (R₁ group) | 8 |
| 7**a** (X = O, R₁ = 2-furany l, Y = phenyl ) | DMS O-*d*₆/40 0 MHz | 6.23 (d, *J* = 6.4 Hz, 1H) | 4.62-4.58 (m, 1H) | 4.34-4.30 (m, 1H) | 3.66-3.61( m, 1H) | 4.75 (dd, 10.9, 6.8 Hz, 1H), 4.51 (dd, *J* | 7.99 (merge d dd, *J*₁ = *J*₂ = 7.3 Hz, 2H), 7.68 (merge | 8.14 (s, 1H) | 7.79-7.77 (m, 1H), 6.63-6.59 (m, 2H) | 7.91 (s, 1H) |
| | | | | | | = 11.0, 6.8 Hz, 1H) | d dd, *J*₁ = *J*₂ = 7.3 Hz, 1H), 7.53 (merge d dd, *J*₁ = *J*₂ = 7.7 Hz, 2H) | | | |
| **7b**(X = O, = ethyny l, Y = phenyl ) | CD₃O D/40 0 MHz | 6.16 (d, *J* = 5.5 Hz, 1H) | 4.50-4.46 (m, 1H) | 4.35-4.31 (m, 1H) | 3.79-3.74 (m, 1H) | 4.80-4.72 (m, 1H), 4.59-4.51 (m, 1H) | 8.09-8.03 (m, 2H), 7.63-7.60 (m, 1H), 7.52-7.49 (m, 2H) | 8.10 (s, 1H) | 3.70 (s, 1H) | 7.70 (s, 1H) |
| **7c**(X = O, R₁ = ethyny l, Y = *p-*chloro phenyl ) | DMS O-*d*₆/40 0 MHz | 6.08 (d, *J* = 6.1 Hz, 1H) | 4.53-4.51 (m, 1H) | 4.24-4.20 (m, 1H) | 3.60-3.56 (m, 1H) | 4.71-4.68 (m, 1H), 4.50-4.42 (m, 1H) | 7.96-7.94 (m, 2H), 7.60-7.58 (m, 2H) | 8.09 (s, 1H) | 4.25 (s, 1H) | 7.87 (s, 1H) |
| **7d**(X = O, R₁ = ethyny l, Y = *p-*metho xyphe nyl) | CD₃O D/40 0 MHz | 6.14 (d, *J* = 5.6 Hz, 1H) | 4.50-4.46 (m, 1H) | 4.34-4.31 (m, 1H) | 3.76-3.72 (m, 1H) | 4.76-4.71 (m, 1H), 4.54-4.50 (m, 1H) | 8.02-7.98 (m, 2H), 7.03-6.99 (m, 2H), 3.85 (s, 3H) | 8.10 (s, 1H) | 3.70 (s, 1H) | 7.67 (s, 1H) |
| **7e**(X = O, R₁ = ethyny l, Y 2,6-dimet hylphe nyl) | CD₃O D/40 0 MHz | 6.15 (d, *J* = 4.8 Hz, 1H) | 4.44-4.41 (m, 1H) | 4.29-4.25 (m, 1H) | 3.77-3.72 (m, 1H) | 4.75-4.70 (m, 1H), 4.61-4.56 (m, 1H) | 7.23-7.18 (m, 1H), 7.08-7.05 (m, 2H), 2.31 (s, 6H) | 8.10 (s, 1H) | 3.67 (s, 1H) | 7.56 (s, 1H) |
| **7f**(X = O, R₁ = ethyny l, Y = isonic otinyl) | DMS O-*d*₆/40 0 MHz | 6.11 (d, *J* = 6.1 Hz, 1H) | 4.55-4.53 (m, 1H) | 4.30-4.28 (m, 1H) | 3.65-3.61 (m, 1H) | 4.79-4.75 (m, 1H), 4.56-4.54 (m, 1H) | 8.84-8.82 (m, 2H), 7.86-7.85 (m, 2H) | 8.13 (s, 1H) | 4.29 (s, 1H) | 7.92 (s, 1H) |
| **7g**(X = O, R₁ = CN, Y = phenyl ) | CD₃O D/40 0 MHz | 6.18 (d, *J* = 5.4 Hz, 1H) | 4.55-4.42 (m, 1H) | 4.38-4.35 (m, 1H) | 3.76-3.70 (m, 1H) | 4.92-4.79 (m, 1H), 4.60-4.56 (m, 1H) | 8.0-7.69 (m, 2H), 7.66-7.58 (m, 1H), 7.54-7.47 (m, 2H) | 8.16 (s, 1H) | - | 7.90 (s, 1H) |
| **7h**(X = O, R₁ = CON H₂, Y = phenyl ) | CD₃O D/40 0 MHz | 6.17 (d, *J* = 5.3 Hz, 1H) | 4.53-4.39 (m, 1H) | 4.36-4.32 (m, 1H) | 3.79-3.72 (m, 1H) | 4.90-4.78 (m, 1H), 4.62-4.55 (m, 1H) | 8.05-7.73 (m, 2H), 7.67-7.56 (m, 1H), 7.55-7.45 (m, 2H) | 8.15 (s, 1H) | - | 7.89 (s, 1H) |
| **8a**(X = NH, R₁ = 2-furany l, Y = phenyl ) | DMS O-*d*₆/40 0 MHz | 6.17 (d, *J* = 7.2 Hz, 1H) | 4.56-4.50 (m, 1H) | 4.20-4.16 (m, 1H) | 3.44-3.38 (m, 1H) | 3.78-3.70 (m, 1H), 3.55-3.48 (m, 1H) | 7.85-7.82 (m, 2H), 7.52-7.48 (m, 1H), 7.46-7.40 (m, 2H) | 8.09 (s, 1H) | 7.75-7.74 (m, 1H), 6.66-6.64 (m, 1H), 6.59-6.57 (m, 1H) | 7.92 (s, 1H) |
| **8b**(X = NH, R₁ = ethyny l, Y = phenyl ) | CD₃O D/40 0 MHz | 6.17 (d, *J* = 5.6 Hz, 1H) | 4.54-4.51 (m, 1H) | 4.24-4.21 (m, 1H) | 3.67-3.64 (m, 1H) | 3.89-3.83 (m, 1H), 3.75-3.70 (m, 1H) | 7.86-7.82 (m, 2H), 7.56-7.52 (m, 1H), | 8.08 (s, 1H) | 3.73 (s, 1H) | 7.79 (s, 1H) |
| | | | | | | | 7.49-7.44 (m, 2H) | | | |
| **8c**(X = NH, R₁ = ethyny l, Y = *p-*metho xyphe nyl) | CD₃O D/40 0 MHz | 6.17 (d, *J* = 6.0 Hz, 1H) | 4.54-4.51 (m, 1H) | 4.23-4.21 (m, 1H) | 3.65-3.62 (m, 1H) | 3.87-3.81 (m, 1H), 3.73-3.70 (m, 1H) | 7.84-7.81 (m, 2H), 7.01-6.98 (m, 2H), 3.85 (s, 3H) | 8.09 (s, 1H) | 3.73 (s, 1H) | 7.78 (s, 1H) |
| **8d**(X = NH, R₁ = CN, Y = phenyl ) | CD₃O D/40 0 MHz | 6.16 (d, *J* = 5.7 Hz, 1H) | 4.50-4.46 (m, 1H) | 4.27-4.19 (m, 1H) | 3.65-3.62 (m, 1H) | 3.88-3.82 (m, 1H), 3.74-3.68 (m, 1H) | 7.85-7.81 (m, 2H), 7.55-7.51 (m, 1H), 7.47-7.43 (m, 2H) | 8.10 (s, 1H) | - | 7.80 (s, 1H) |
| **8e**(X = NH, R₁ = CON H₂, Y = phenyl ) | CD₃O D/40 0 MHz | 6.15 (d, *J* = 5.6 Hz, 1H) | 4.52-4.47 (m, 1H) | 4.25-4.18 (m, 1H) | 3.63-3.61 (m, 1H) | 3.89-3.85 (m, 1H), 3.75-3.66 (m, 1H) | 7.86-7.79 (m, 2H), 7.56-7.50 (m, 1H), 7.46-7.42 (m, 2H) | 8.09 (s, 1H) | - | 7.78 (s, 1H) |

**[Table 4]**

| Compound | HRMS, *m*/*z* [M + H]⁺ | Molecular formula |
|---|---|---|
| **7a**(X = O, R₁ = 2-furanyl, Y = phenyl) | 453.1244 | C₂₂H₂₁N₄O₅S |
| **7b**(X = O, R₁ = ethynyl, Y = phenyl) | 411.1129 | C₂₀H₁₉N₄O₄S |
| **7c**(X = O, R₁ = ethynyl, Y = *p*-chlorophenyl) | 445.0737 | C₂₀H₁₈ClN₄O₄S |
| **7d**(X = O, R₁ = ethynyl, Y = *p*-methoxyphenyl) | 441.1231 | C₂₁H₂₁N₄O₅S |
| **7e**(X = O, R₁ = ethynyl, Y = 2,6-dimethylphenyl) | 439.1441 | C₂₂H₂₃N₄O₄S |
| **7f**(X = O, R₁ = ethynyl, Y = isonicotinyl) | 412.1082 | C₁₉H₁₈N₅O₄S |
| **7g**(X = O, R₁ = CN, Y = phenyl) | 412.1035 | C₁₉H₁₈N₅O₄S |
| **7h**(X = O, R₁ = CONH₂, Y = phenyl) | 430.1140 | C₁₉H₂₀N₅O₅S |
| **8a**(X = NH, R₁ = 2-furanyl, Y = phenyl) | 452.1392 | C₂₂H₂₂N₅O₄S |
| **8b**(X = NH, R₁ = ethynyl, Y = phenyl) | 410.1277 | C₂₀H₂₀N₅O₃S |
| **8c**(X = NH, R₁ = ethynyl, Y = *p*-methoxyphenyl) | 440.1401 | C₂₁H₂₂N₅O₄S |
| **8d**(X = NH, R₁ = CN, Y = phenyl) | 411.1195 | C₁₉H₁₉N₆O₃S |
| **8e**(X = NH, R₁ = CONH₂, Y = phenyl) | 429.1300 | C₁₉H₂₁N₆O₄S |

### Preparation Example 6: Synthesis of Compounds 9a to 9c, 10a to 10c, 11ai to 11aiii and 11b

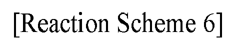

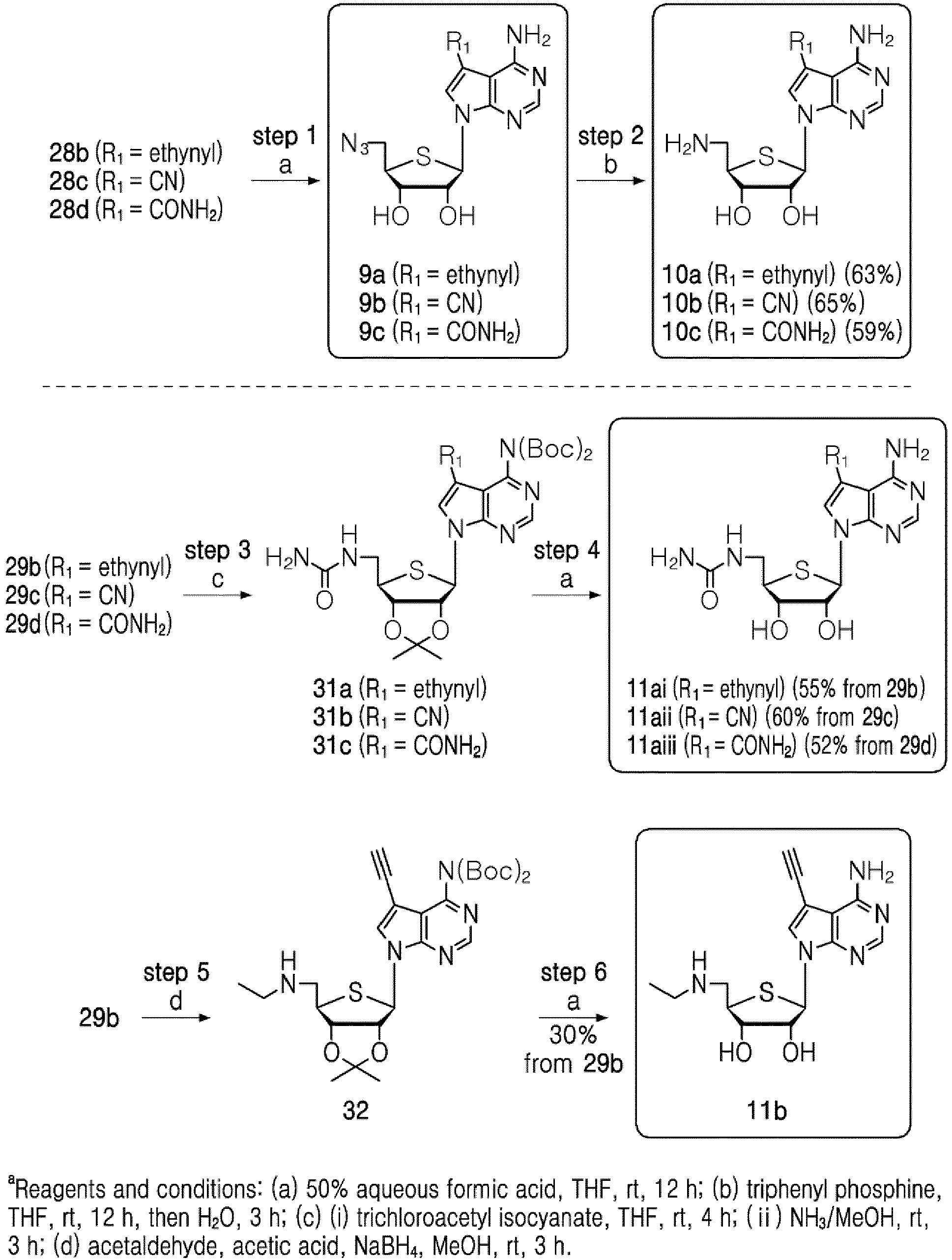

### Preparation Example 6-1: Synthesis of Compounds 9a to 9c

Compounds 9a to 9c were synthesized from Compounds 28b to 28d, respectively, by the known synthetic method (H).

### Preparation Example 6-2: Synthesis of Compounds 10a to 10c

After Compounds 9a to 9c (1 eq.) were dissolved in tetrahydrofuran (5.6 mL/mmol), triphenylphosphine (2 eq.) was added thereto, and then the solution was stirred at room temperature for 12 hours. After water was added to the reaction solution, the resulting mixture was stirred at room temperature for another 3 hours. Volatile materials were evaporated, and the residue was purified by silica gel column chromatography to synthesize Compounds 10a to 10c in a yield of 63%, 65%, and 59%, respectively.

### Preparation Example 6-3: Synthesis of Compounds 31a to 31c

While stirring solutions of Compounds 29b and 29d (1 eq.) dissolved in anhydrous tetrahydrofuran (6.6 mL/mmol), trichloroacetyl isocyanate (1 eq.) was added dropwise thereto, and the reaction solution was stirred at room temperature for 4 hours. After the reaction was terminated with water, extraction was performed three times with ethyl acetate. The organic solvent layer was dried, and then filtered under reduced pressure, and a crude residue obtained by performing concentration under reduced pressure was used in the next reaction. After methanol saturated with ammonia (5 mL/mmol) was added to the crude compound, the resulting mixture was stirred at room temperature for 3 hours. After volatile materials were evaporated, crude Compounds 31a to 31c were used in the next reaction.

### Preparation Example 6-4: Synthesis of Compounds 11ai to 11aiii

Compounds 11ai to 11aiii were synthesized from Compounds 31a to 31c in a yield of 55%, 60%, and 52% (2 steps), respectively, by the known synthetic method (H).

### Preparation Example 6-5: Synthesis of tert-butyl (tertbutoxycarbonyl)(7-((3aR,4R,6R,6aS)-6-((ethylamino)methyl)-2,2-dimethyltetrahydrothieno[3,4-d][1,3]dioxol-4-yl)-5-ethynyl-7H-pyrrolo[2,3-d]pyrimidin-4-yl)carbamate (Compound 32)

After Compound 29b (0.05 g, 0.09 mmol) and acetaldehyde (0.08 mL) were dissolved in methanol (0.35 mL), sodium borohydride and acetic acid (4 µL) were added dropwise thereto. After the reaction solution was stirred at room temperature for 3 hours, the reaction was terminated with water. Thereafter, extraction was performed three times with ethyl acetate. The organic solvent layer was dried over MgSO₄, and then subjected to filtration under reduced pressure and concentration under reduced pressure to synthesize crude Compound 32, and the compound was used in the next reaction without any purification process.

### Preparation Example 6-6: Synthesis of (2R,3R,4S,5R)-2-(4-amino-5-ethynyl-7H-pyrrolo[2,3-d]pyrimidin-7-yl)-5-((ethylamino)methyl)tetrahydrothiophene-3,4-diol (Compound 11b)

Compound 11b was synthesized from Compound 32 in a yield of 30% (2 steps) by the known synthetic method (H).

### Preparation Example 7: Synthesis of Compounds 12a and 12b

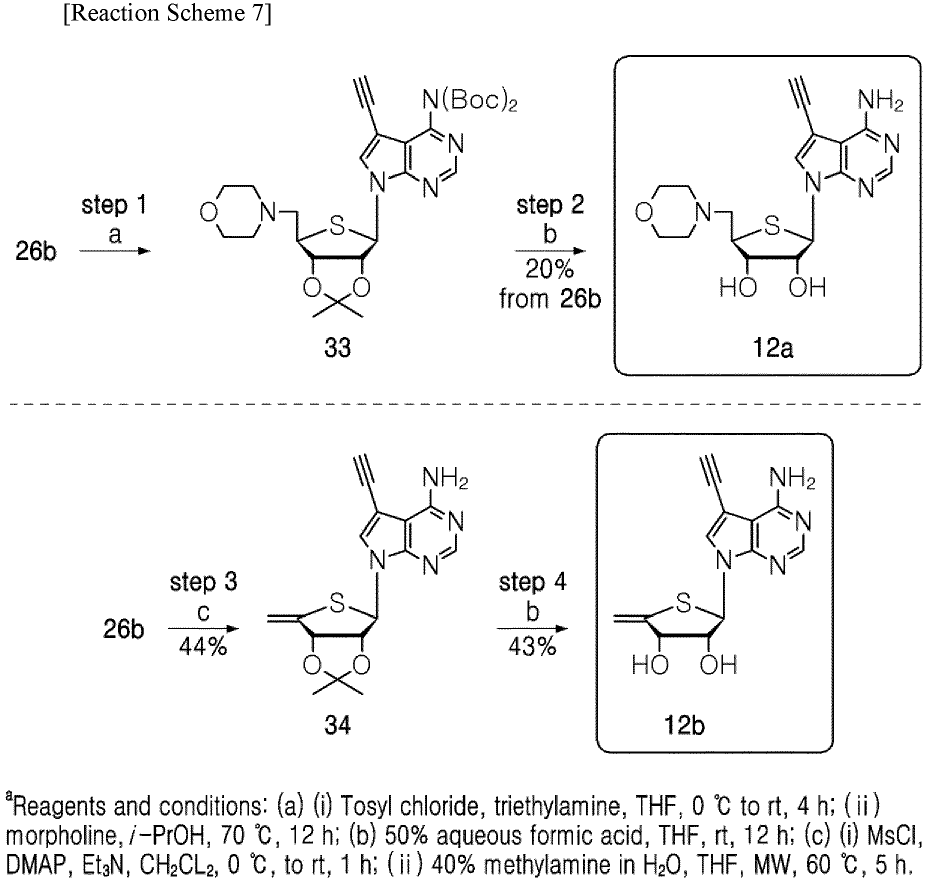

### Preparation Example 7-1: Synthesis of tert-butyl (tert-butoxycarbonyl)(7-((3aR,4R,6R,6aS)-2,2-dimethyl-6-(morpholinomethyl)tetrahydrothieno[3,4-d][1,3]dioxol-4-yl)-5-ethynyl-7H-pyrrolo[2,3-d]pyrimidin-4-yl)carbamate (Compound 33)

Compound 26b (0.15 g, 0.27 mmol) dissolved in tetrahydrofuran and triethylamine (0.2 mL) were added to a solution of 4-toluenesulfonyl chloride (0.057 g, 0.30 mmol) dissolved in tetrahydrofuran (0.3 mL) at 0°C. The reaction solution was stirred at room temperature for 4 hours, and the reaction was terminated with water. After extraction was performed three times with ethyl acetate, a collected organic solvent layer was dried over MgSO₄, filtered under reduced pressure, and concentrated under reduced pressure, and a crude compound was used in the next reaction without any purification process. After the crude toluenesulfonyl compound was dissolved in isopropyl alcohol (0.6 mL), morpholine (0.02 g, 0.23 mmol) was added thereto, and the resulting mixture was refluxed at 70°C for 12 hours. After filtration under reduced pressure, the resulting product was used in the next reaction without any purification process.

### Preparation Example 7-2: Synthesis of (2R,3R,4S,5R)-2-(4-amino-5-ethynyl-7H-pyrrolo[2,3-d]pyrimidin-7-yl)-5-(morpholinomethyl)tetrahydrothiophene-3,4-diol (Compound 12a)

Compound 12a was synthesized from Compound 33 in a yield of 20% (2 steps) by the known synthetic method (H).

### Preparation Example 7-3: Synthesis of 7-((3aR,4R,6aS)-2,2-dimethyl-6-methylenetetrahydrothieno[3,4-d][1,3]dioxol-4-yl)-5-ethynyl-7H-pyrrolo[2,3-d]pyrimidine-4-amine (Compound 34)

4-dimethylaminopyridine (0.0004 g, 0.003 mmol) and triethylamine (0.11 mL, 0.82 mmol) were added to a solution of Compound 26b (0.15 g, 0.27 mmol) dissolved in dichloromethane (2.2 mL), and the solution was cooled to 0°C. Methanesulfonyl chloride (0.03 mL, 0.41 mmol) was added dropwise to this solution, and the reaction solution was stirred at room temperature for 1 hour. The reaction was terminated with a saturated sodium bicarbonate solution, and extraction was performed three times with dichloromethane. A collected organic solvent layer was washed with brine, dried over MgSO₄, filtered under reduced pressure, and concentrated under reduced pressure to obtain a crude methanesulfonyl compound. Tetrahydrofuran (2.5 mL) and a 40% aqueous methylamine solution (2 mL) were added to the crude methanesulfonyl compound contained in a microwave vial, and the resulting mixture was heated at 60°C for 5 hours. Volatile materials were evaporated, and the residue was purified by silica gel column chromatography to obtain Compound 34 (39.6 mg, 44%).

¹H NMR (CD₃OD, 400 MHz): *δ* 8.11 (s, 1H), 7.35 (s, 1H), 6.33 (s, 1H), 5.52 (s, 1H), 5.46 (merged dd, *J*₁ = *J*₂ = 4.8 Hz, 1H), 5.32 (s, 1H), 4.96 (dd, *J* = 4.8, 0.8 Hz, 1H), 3.73 (s, 1H), 1.50 (s, 3H), 1.33 (s, 3H); HRMS (ESI-Q-TOF) *m*/*z* [M + H]⁺ for C₁₆H₁₇N₄O₂S calculated 329.1067, found 329.1064.

### Preparation Example 7-4: Synthesis of (2R,3R,4S)-2-(4-amino-5-ethynyl-7H-pyrrolo[2,3-d]pyrimidin-7-yl)-5-methylenetetrahydrothiophene-3,4-diol (Compound 12b)

Compound 12b was synthesized from Compound 34 in a yield of 43% by the known synthetic method (H).

The following Tables 5 and 6 summarize the ¹H NMR and high resolution mass spectrometry (HRMS) data of compounds represented by the following Chemical Formulae III and IV, respectively.

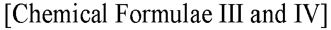

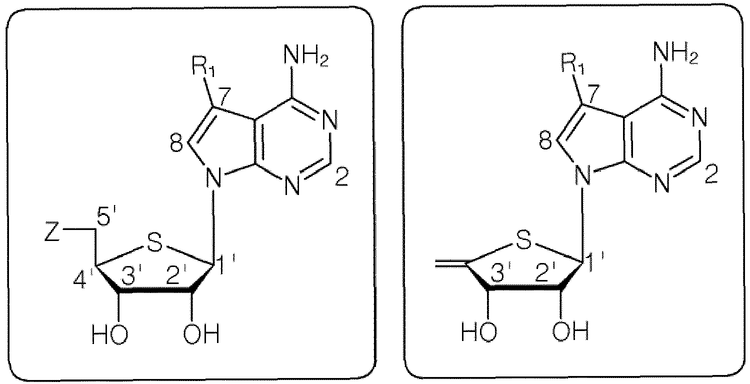

**[Table 5]**

| Compou nd | NM R Solv ent/F requ ency | ¹H NMR data (Chemical shift, *δ* ppm) | | | | | | | | |
|---|---|---|---|---|---|---|---|---|---|---|
| | | 1' | 2' | 3' | 4' | 5' | Z group/ =CH₂ | 2 | 7 (R₁ group ) | 8 |
| 9a (R₁ = ethynyl, Z = N₃) (Compo und III) | DM SO*d*₆/50 0 MHz | 6.10 (d, *J* = 6.8 Hz, 1H) | 4.52-4.48 (m, 1H) | 4.09-4.07 (m, 1H) | 3.38-3.34 (m, 1H) | 3.89-3.84 (m, 1H), 3.78-3.73 (m, 1H) | - | 8.12 (s, 1H) | 4.30 (s, 1H) | 7.94 (s, 1H) |
| 9b(R₁ = CN, Z = N₃) (Compo und III) | DM SO*d*₆/50 0 MHz | 6.11 (d, *J* = 6.5 Hz, 1H) | 4.55-4.49 (m, 1H) | 4.11-4.08 (m, 1H) | 3.36-3.34 (m, 1H) | 3.87-3.85 (m, 1H), 3.79-3.72 (m, 1H) | - | 8.15 (s, 1H) | - | 7.96 (s, 1H) |
| 9c(R₁ = CONH₂, Z = N₃) (Compo und III) | DM SO*d*₆/50 0 MHz | 6.09 (d, *J* = 6.6 Hz, 1H) | 4.54-4.47 (m, 1H) | 4.09-4.05 (m, 1H) | 3.35-3.33 (m, 1H) | 3.86-3.83 (m, 1H), 3.80-3.71 (m, 1H) | - | 8.13 (s, 1H) | - | 7.95 (s, 1H) |
| 10a(R₁ = ethynyl, Z = NH₂) | DM SO*d*₆/40 0 | 6.07 (d, *J* = 6.7 Hz, | 4.44-4.40 (m, 1H) | 4.14-4.11 (m, 1H) | 3.26-3.21 (m, 1H) | 3.03-2.97 (m, 1H), | 4.22 (t, 2H) | 8.12 (s, 1H) | 4.30 (s, 1H) | 7.94 (s, 1H) |
| (Compo und III) | MHz | 1H) | | | | 2.85-2.78 (m, 1H) | | | | |
| 10b(R₁ = CN, Z = NH₂) (Compo und III) | DM SO*d*₆/40 0 MHz | 6.08 (d, *J* = 6.9 Hz, 1H) | 4.45-4.42 (m, 1H) | 4.16-4.13 (m, 1H) | 3.25-3.20 (m, 1H) | 3.05-2.96 (m, 1H), 2.84-2.75 (m, 1H) | 4.21 (t, 2H) | 8.14 (s, 1H) | - | 7.95 (s, 1H) |
| 10c(R₁ = CONH₂, Z = NH₂) (Compo und III) | DM SO*d*₆/40 0 MHz | 6.06 (d, *J* = 6.8 Hz, 1H) | 4.49-4.43 (m, 1H) | 4.15-4.12 (m, 1H) | 3.24-3.21 (m, 1H) | 3.07-2.95 (m, 1H), 2.86-2.77 (m, 1H) | 4.20 (t, 2H) | 8.13 (s, 1H) | - | 7.94 (s, 1H) |
| 11ai(R₁ = ethynyl, Z = NHCON H₂) (Compo und III) | CD₃ OD/ 400 MHz | 6.15 (d, *J* = 6.4 Hz, 1H) | 4.47-4.44 (m, 1H) | 4.15-4.12 (m, 1H) | 3.48-3.42 (m, 1H) | 3.06-3.00 (m, 2H) | - | 8.09 (s, 1H) | 3.72 (s, 1H) | 7.77 (s, 1H) |
| 11aii(R₁ = CN, Z NHCON H₂) (Compo und III) | CD₃ OD/ 400 MHz | 6.16 (d, *J* = 6.2 Hz, 1H) | 4.46-4.42 (m, 1H) | 4.17-4.13 (m, 1H) | 3.46-3.41 (m, 1H) | 3.05-3.00 (m, 2H) | - | 8.10 (s, 1H) | - | 7.78 (s, 1H) |
| 11aiii(R₁ CONH₂, Z = NHCON H₂) (Compo und III) | CD₃ OD/ 400 MHz | 6.15 (d, *J* = 6.3 Hz, 1H) | 4.45-4.41 (m, 1H) | 4.16-4.11 (m, 1H) | 3.45-3.40 (m, 1H) | 3.07-3.01 (m, 2H) | - | 8.09 (s, 1H) | - | 7.76 (s, 1H) |
| 11b (Z = NHCH₂ CH₃) (Compo und III) | CD₃ OD/ 400 MHz | 6.10 (d, *J* = 5.2 Hz, 1H) | 4.42-4.40 (m, 1H) | 4.16-4.12 (m, 1H) | 3.50-3.46 (m, 1H) | 2.86-2.79 (m, 1H), 2.75-2.70 (m, | 2.59-2.50 (m, 2H), 1.11-1.09 (m, | 8.12 (s, 1H) | 3.73 (s, 1H) | 7.86 (s, 1H) |
| | | | | | | 1H) | 3H) | | | |
| 12a (Z = morpholi ne) (Compo und III) | CD₃ OD/ 400 MHz | 6.09 (d, *J* = 4.4 Hz, 1H) | 4.33-4.30 (m, 1H) | 4.18-4.14 (m, 1H) | 3.64-3.58 (m, 1H) | 2.95-2.90 (m, 1H), 2.76-2.70 (m, 1H) | 3.72-3.68 (m, 4H), 2.62-2.52 (m, 4H) | 8.12 (s, 1H) | 3.74 (s, 1H) | 7.85 (s, 1H) |
| 12b (Compo und IV) | CD₃ OD/ 400 MHz | 6.43 (d, *J* = 6.8 Hz, 1H) | 4.60-4.58 (m, 1H) | 4.46-4.42 (m, 1H) | - | - | 5.41 (merg ed dd, *J*₁ = *J*₂ = 0.8 Hz, 1H), 5.11 (s, 1H) | 8.13 (s, 1H) | 3.74 (s, 1H) | 7.67 (s, 1H) |

**[Table 6]**

| Compound | HRMS, *m*/*z* [M + H]⁺ | Molecular formula |
|---|---|---|
| 9a(R₁ = ethynyl, Z = N₃) (Compound III) | 332.0923 | C₁₃H₁₄N₇O₂S |
| 9b(R₁ = CN, Z = N₃) (Compound III) | 333.0837 | C₁₂H₁₃N₈O₂S |
| 9c(R₁ = CONH₂, Z = N₃) (Compound III) | 351.0943 | C₁₂H₁₅N₈O₃S |
| 10a(R₁ = ethynyl, Z = NH₂) (Compound III) | 306.1031 | C₁₃H₁₆N₅O₂S |
| 10b(R₁ = CN, Z = NH₂) (Compound III) | 307.0932 | C₁₂H₁₅N₆O₂S |
| 10c(R₁ = CONH₂, Z = NH₂) (Compound III) | 325.1038 | C₁₂H₁₇N₆O₃S |
| 11ai(R₁ = ethynyl, Z = NHCONH₂) (Compound III) | 349.1092 | C₁₄H₁₇N₆O₃S |
| 11aii(R₁ = CN, Z = NHCONH₂) (Compound III) | 350.0991 | C₁₃H₁₆N₇O₃S |
| 11aiii(R₁ = CONH₂, Z = NHCONH₂) (Compound III) | 368.1096 | C₁₃H₁₈N₇O₄S |
| 11b (Z = NHCH₂CH₃) (Compound III) | 334.1299 | C₁₅H₂₀N₅O₂S |
| 12a (Z = morpholine) (Compound III) | 616.2805 | C₃₀H₄₂N₅O₇S |
| 12b (Compound IV) | 289.0759 | C₁₃H₁₃N₄O₂S |

### Preparation Example 8: Synthesis of Compounds 13a to 13g

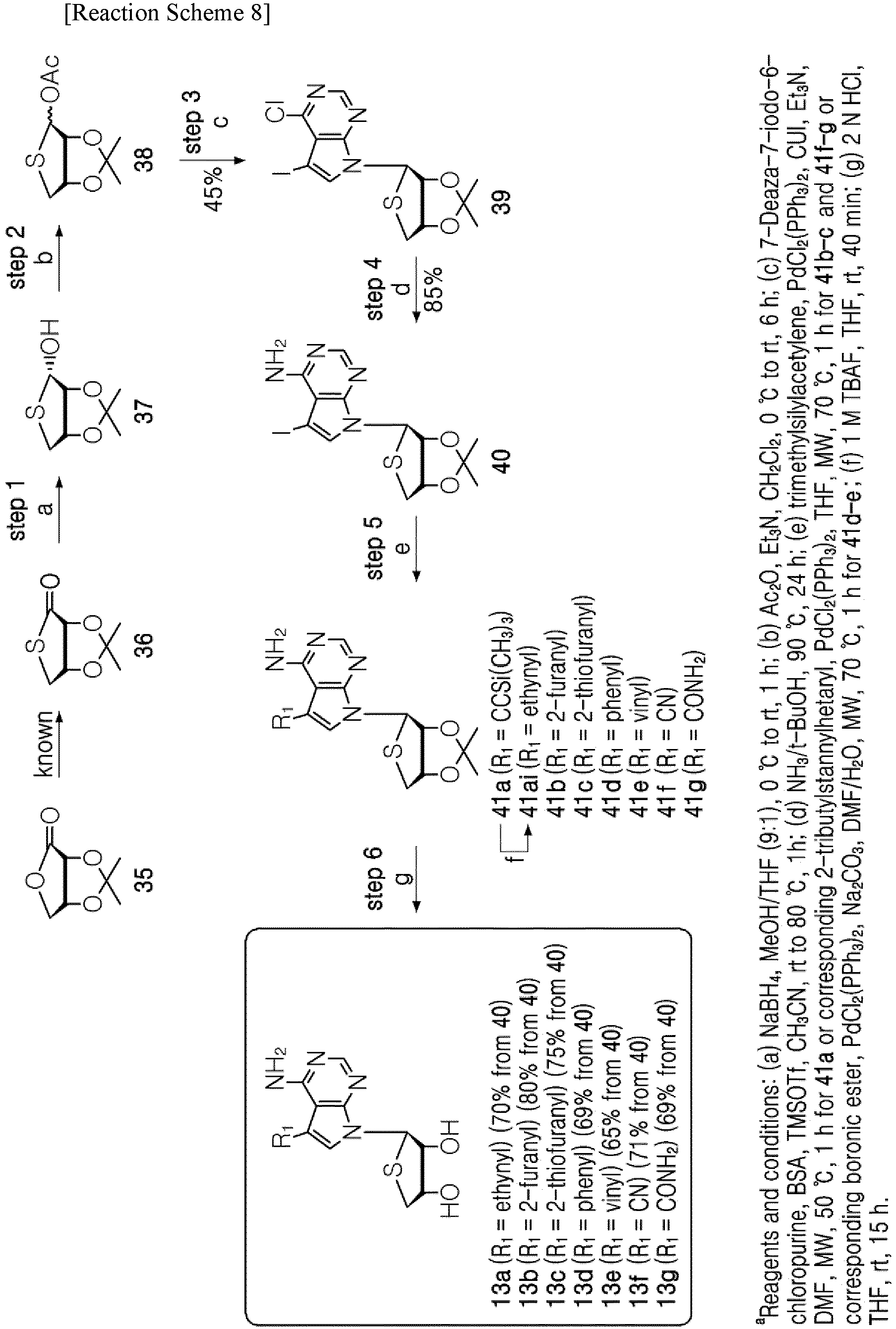

### Preparation Example 8-1: Synthesis of (3aR,4S,6aS)-2,2-dimethyltetrahydrothieno[3,4-d][1,3]dioxol-4-ol (Compound 37)

After sodium borohydride (0.21 g, 5.74 mmol) was added dropwise to Compound 36 (1 g, 5.74 mmol) dissolved in methanol/tetrahydrofuran (30 mL, 9:1) at 0°C, the resulting mixture was stirred at the same temperature for 30 minutes, and then stirred at room temperature for 30 minutes. After the reaction was terminated with water, an aqueous solution layer was extracted three times with ethyl acetate. A collected organic solvent layer was dried over MgSO₄, filtered under reduced pressure, and concentrated under reduced pressure to synthesize crude Compound 37. This compound was pure enough to be immediately used in the next reaction.

### Preparation Example 8-2: Synthesis of (3aR,6aS)-2,2-dimethyltetrahydrothieno[3,4-d][1,3]dioxol-4-yl acetate (Compound 38)

Triethylamine (21.5 mL, 154.62 mmol) was added to a solution of Compound 37 (5.45 g, 30.92 mmol) dissolved in dichloromethane (54 mL) under stirring, and then acetic anhydride (5.8 mL, 61.85 mmol) was added dropwise thereto at 0°C. The reaction solution was stirred at room temperature for 6 hours, the reaction was terminated with a saturated sodium bicarbonate solution, and then extraction was performed three times with ethyl acetate. A collected organic solvent layer was dried, filtered under reduced pressure, and concentrated under reduced pressure. This compound was pure enough to be immediately used in the next reaction.

### Preparation Example 8-3: Synthesis of 4-chloro-7-((3aR,4R,6aS)-2,2-dimethyltetrahydrothieno[3,4-d][1,3]dioxol-4-yl)-5-iodo-7H-pyrrolo[2,3-d]pyrimidine (Compound 39)

N,O-bis(trimethylsilyl)acetamide (BSA, 7.2 mL, 29.78 mmol) was added to 4-chloro-5-iodo-7*H*-pyrrolo[2,3-*d*]pyrimidine (7.5 g, 27.07 mmol) under stirring in anhydrous acetonitrile (90 mL) under a nitrogen environment, and the resulting mixture was stirred at room temperature for 10 minutes until the resulting mixture became a homogeneous solution. Anhydrous acetonitrile (65 mL), in which Compound 38 (6.5 g, 29.78 mmol) was dissolved, was added to this clear solution was added, and then trimethylsilyl trifluoromethanesulfonate (4.3 mL, 24.36 mmol) was added dropwise thereto. The reaction solution was stirred at room temperature for 15 minutes and transferred under a condition in which it had been heated to 80°C in advance. After stirring at 80°C for 1 hour, the reaction solution was cooled to room temperature. The reaction solution was then diluted with ethyl acetate. The organic solvent layer was washed with saturated sodium bicarbonate and dried over MgSO₄. Moreover, the organic solvent layer was filtered under reduced pressure and concentrated under reduced pressure, and the residue was purified by silica gel column chromatography to obtain Compound 39 (5.28 g, 45%).

¹H NMR (CD₃OD, 500 MHz): *δ* 8.58 (s, 1H), 7.83 (s, 1H), 6.12 (s, 1H), 5.32 (t, *J* = 4.8 Hz, 1H), 5.22 (merged dd, *J*₁ = *J*₂ = 5.3 Hz, 1H), 3.70 (dd, *J* = 12.9, 4.4 Hz, 1H), 3.12 (merged dd, *J*₁ = *J*₂ = 13 Hz, 1H), 1.53 (s, 3H), 1.32 (s, 3H); HRMS (ESI-Q-TOF) *mlz* [M + H]⁺ for C₁₃H₁₄ClIN₃O₂S calculated 437.9534, found 437.9523.

### Preparation Example 8-4: Synthesis of 7-((3aR,4R,6aS)-2,2-dimethyltetrahydrothieno [3,4-d][1,3]dioxol-4-yl)-5-iodo-7H-pyrrolo[2,3-d]pyrimidine-4-amine (Compound 40)

After Compound 39 (3.5 g, 7.99 mmol) was dissolved in tertiary butyl alcohol (35 mL) saturated with ammonia, the resulting solution was put into a stainless steel bomb and transferred under a condition in which it had been heated to 90°C in advance, and then stirred at the same temperature for 24 hours. After the steel bomb containing the reaction solution was cooled to room temperature, the reaction solution was concentrated under reduced pressure to evaporate the solvent. The residue was purified by silica gel to obtain Compound 40 (2.84g, 85%).

¹H NMR (CDCl₃, 500 MHz): *δ* 8.25 (s, 1H), 7.12 (s, 1H), 5.89 (s, 1H), 5.65 (brs, 2H), 5.20-5.19 (m, 1H), 5.15 (merged dd, *J*₁ = *J*₂ = 5.4 Hz, 1H), 3.63 (dd, *J* = 12.8, 4.4 Hz, 1H), 3.15 (merged dd, *J*₁ = *J*₂ = 12.8 Hz, 1H), 1.56 (s, 3H), 1.32 (s, 3H); HRMS (ESI-Q-TOF) *m*/*z* [M + H]⁺ for C₁₃H₁₆IN₄O₂S calculated 419.0033, found 419.0031.

### Preparation Example 8-5: Synthesis of Compounds 41a to 41g

Compound 41a was synthesized from Compound 40 by the known synthetic method (D). Compound 41ai was synthesized from Compound 41a by the known synthetic method (E). Compounds 41b and 41c were synthesized from Compound 40 by the known synthetic method (A). Compounds 41d and 41e were synthesized from Compound 40 by the known synthetic method (C). Compounds 41f and 41g were synthesized from Compound 40 by the known synthetic method (A). Compounds 41a to 41g were used in the next reaction without any purification process.

### Preparation Example 8-6: Synthesis of Compounds 13a to 13g

Compounds 13a to 13g were synthesized from Compounds 41ai to 41g in a yield of 70%, 80%, 75%, 69%, 65%, 71%, and 69% (2 steps), respectively, by the known synthetic method (F).

The following Tables 7 and 8 summarize the ¹H NMR and high resolution mass spectrometry (HRMS) data of compounds represented by the following Chemical Formula V, respectively.

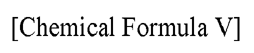

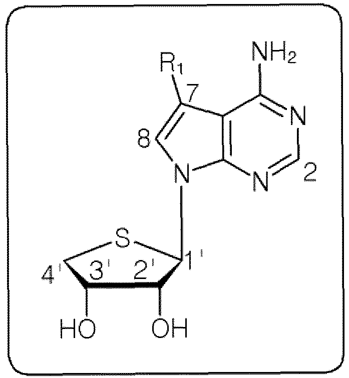

**[Table 7]**

| Compou nd | NMR Solven t/Frequ ency | ¹H NMR data (Chemical shift, *δ* ppm) | | | | | | |
|---|---|---|---|---|---|---|---|---|
| | | 1' | 2' | 3' | 4' | 2 | 7 (R₁ group) | 8 |
| 13a (R₁ = ethynyl) | CD₃O D/400 MHz | 6.20 (d, *J* = 6.4 Hz, 1H) | 4.40-4.38 (m, 1H) | 4.43-4.41 (m, 1H) | 3.48-3.44 (m, 1H), 2.91-2.88 (m, 1H) | 8.10 (s, 1H) | 3.73 (s, 1H) | 7.76 (s, 1H) |
| 13b(R₁ = 2-furanyl) | CD₃O D/400 MHz | 6.29 (d, *J* = 6.7 Hz, 1H) | 4.43-4.40 (m, 1H) | 4.49-4.46 (m, 1H) | 3.47-3.46 (m, 1H), 2.93-2.90 (m, 1H) | 8.11 (s,1H) | 7.62-7.61 (m, 1H), 6.65-6.64 (m, 1H), 6.56-6.54 (m 1H) | 7.82 (s, 1H) |
| 13c(R₁ = 2-thiofura nyl) | DMSO - *d₆*/500 MHz | 6.20 (d, *J* = 7.3 Hz, 1H) | 4.31-4.29 (m, 1H) | 4.55-4.50 (m, 1H) | 3.42-3.39 (m, 1H), 2.76-2.74 (m, 1H) | 8.15 (s, 1H) | 7.57-7.56 (m, 1H), 7.18-7.15 (m, 2H) | 7.67 (s, 1H) |
| 13d(R₁ phenyl) | CD₃O D/500 MHz | 6.34 (d, *J* = 6.7 Hz, 1H) | 4.47-4.44 (m, 1H) | 4.53-4.51 (m, 1H) | 3.51-3.47 (m, 1H), 2.95-2.92 (m, 1H) | 8.16 (s, 1H) | 7.53-7.48 (m, 4H), 7.41-7.38 (m, 1H) | 7.54 (s, 1H) |
| 13e(R₁ = vinyl) | CD₃O D/400 MHz | 6.23 (d, *J* = 6.4 Hz, 1H) | 4.41-4.39 (m, 1H) | 4.44-4.42 (m, 1H) | 3.46-3.42 (m, 1H), 2.91-2.87 (m, 1H) | 8.15 (s, 1H) | 7.01-6.94 (m, 1H), 5.59-5.54 (m, 1H), 5.24-5.21 (m, 1H) | 7.59 (s, 1H) |
| 13f(R₁ = CN) | CD₃O D/400 MHz | 6.21 (d, *J* = 6.5 Hz, 1H) | 4.42-4.39 (m, 1H) | 4.44-4.43 (m, 1H) | 3.47-3.45 (m, 1H), 2.90-2.89 (m, 1H) | 8.12 (s, 1H) | - | 7.77 (s, 1H) |
| 13g(R₁ = CONH₂) | CD₃O D/400 MHz | 6.20 (d, *J* = 6.6 Hz, 1H) | 4.43-4.40 (m, 1H) | 4.46-4.44 (m, 1H) | 3.46-3.43 (m, 1H), 2.92-2.88 (m, 1H) | 8.11 (s, 1H) | - | 7.77 (s, 1H) |

**[Table 8]**

| Compound | HRMS, *m*/*z* [M + H]⁺ | Molecular formula |
|---|---|---|
| 13a(R₁ = ethynyl) | 277.0756 | C₁₂H₁₃N₄O₂S |
| 13b(R₁ = 2-furanyl) | 319.0868 | C₁₄H₁₅N₄O₃S |
| 13c(R₁ = 2-thienyl) | 335.0639 | C₁₄H₁₅N₄O₂S₂ |
| 13d(R₁ = phenyl) | 329.1075 | C₁₆H₁₇N₄O₂S |
| 13e(R₁ = vinyl) | 279.0906 | C₁₂H₁₅N₄O₂S |
| 13f(R₁ = CN) | 278.0667 | C₁₁H₁₂N₅O₂S |
| 13g(R₁ = CONH₂) | 296.0773 | C₁₁H₁₄N₅O₃S |

### Preparation Example 9: Synthesis of Compound 14

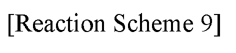

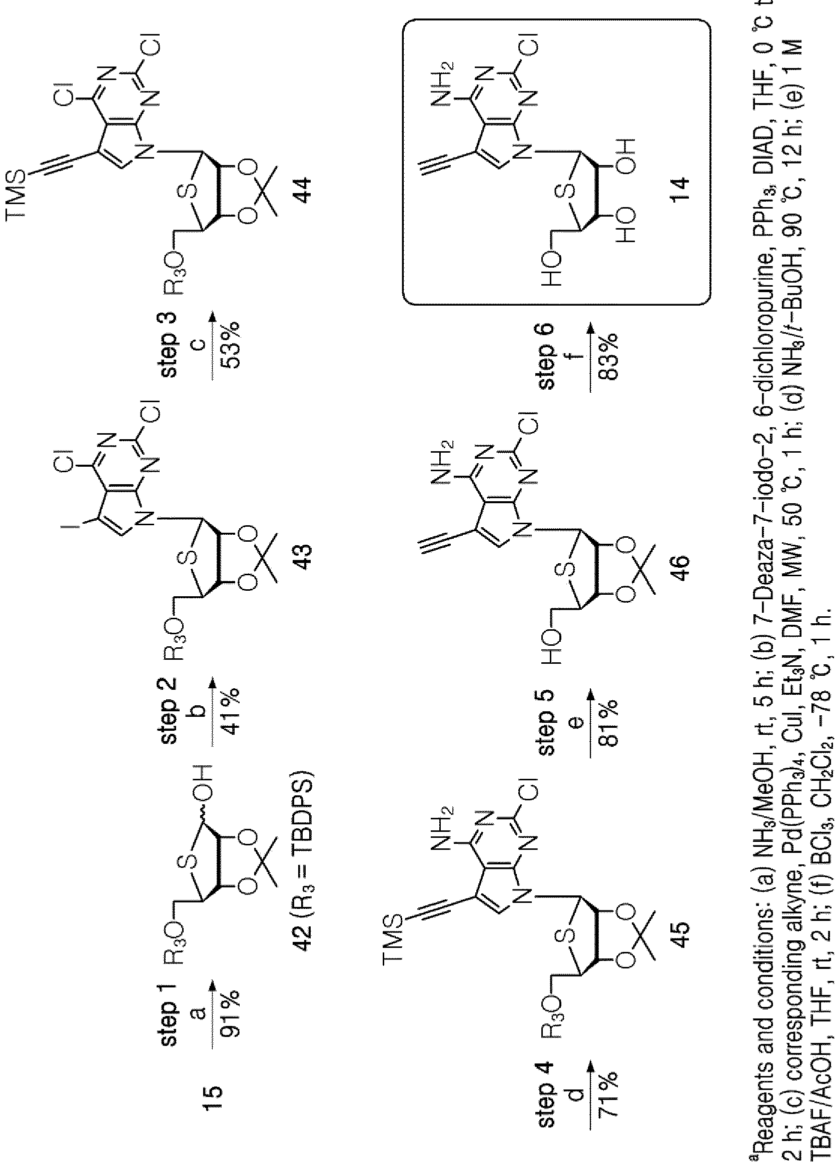

### Preparation Example 9-1: Synthesis of (3aR,6R,6aS)-6-(((tert-butyldiphenylsilyl)oxy)methyl)-2,2-dimethyltetrahydrothieno[3,4-d][1,3]dioxol-4-ol (Compound 42)

Tertiary butyl alcohol saturated with ammonia was added to dichloromethane in which Compound 15 (1.46 g, 3.00 mmol) was dissolved, and the reaction mixture was stirred at room temperature for 4 hours. The reaction mixture was evaporated under reduced pressure conditions. The residue was purified by silica gel column chromatography to obtain Compound 42 (1.22 g, 91%) as a colorless syrup.

¹H NMR (CDCl₃, 400 MHz): *δ* 7.71-7.68 (m, 4H), 7.48-7.41 (m, 6H), 5.29-5.26 (m, 1H), 4.83-4.78 (m, 2H), 4.33-4.31 (brs, 1H), 3.90-3.87 (m, 1H), 3.76-3.72 (m, 1H), 3.55-3.53 (m, 1H).

### Preparation Example 9-2: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-2,4-dichloro-5-iodo-7H-pyrrolo[2,3-d]pyrimidine (Compound 43)

A reaction mixture of Compound 42 (1.14 g, 2.57 mmol), 2,6-dichloro-7-iodo-deazaadenine (1.21 g, 3.86 mmol), and triphenylphosphine (1.35 g, 5.14 mmol) in anhydrous tetrahydrofuran (50 mL) was cooled to 0°C. (*E*)-diisopropyl azodicarboxylate (1.04 g, 5.14 mmol) was added to the reaction mixture, and the reaction temperature was allowed to slowly reach room temperature. The reaction mixture was stirred at room temperature for 2 hours. After neutralization with a saturated aqueous sodium bicarbonate solution, the reaction mixture was distributed in ethyl acetate and water, and extracted three times with ethyl acetate. The organic solvent layer was dried over anhydrous MgSO₄, filtered, and then evaporated. The residue was purified by silica gel column chromatography to obtain Compound 43 (780.9 mg, 41%) as a yellow syrup.

¹H NMR (CDCl₃, 400 MHz): *δ* 7.68-7.64 (m, 4H), 7.59 (s, 1H), 7.44-7.35 (m, 6H), 6.27-6.27 (m, 1H), 4.88-4.82 (m, 2H), 3.95-3.83 (m, 3H), 1.62 (s, 3H), 1.30 (s, 3H), 1.09 (s, 9H).

### Preparation Example 9-3: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-2,4-dichloro-5-((trimethylsilyl)ethynyl)-7H-pyrrolo[2,3-d]pyrimidine (Compound 44)

After a mixture in which DMF (4 mL) and triethylamine (1 mL) were mixed was put into a microwave vial, Compound 43 (153 mg, 0.206 mmol), copper iodide (4.52 mg, 0.041 mmol), and tetrakis(triphenylphosphine) palladium (14.5 mg, 0.020 mmol) were added thereto, and the resulting mixture was degassed with nitrogen gas for 5 minutes. Trimethylsilylacetylene (22.3 mg, 0.22 mmol) was added to the reaction mixture, and the resulting mixture was once again degassed with nitrogen gas for 5 minutes. The reaction mixture was distributed in ethyl acetate and water, extracted three times with ethyl acetate, dried over anhydrous MgSO₄, filtered, and then evaporated. The residue was purified by silica gel column chromatography to obtain Compound 44 (60.2 mg, 53%) as a yellow syrup.

¹H NMR (CDCl₃, 400 MHz): *δ* 7.69-7.65 (m, 5H), 7.46-7.36 (m, 6H), 6.27-6.26 (m, 1H), 4.85-4.82 (m, 2H), 3.93-3.83 (m, 3H), 1.62 (s, 3H), 1.30 (s, 3H), 1.10 (s, 9H), 0.27 (s, 9H). HRMS (FAB) *m*/*z* [M + H]⁺ for C₃₅H₄₁Cl₂N₃O₃SSi₂ found 710.1863.

### Preparation Example 9-4: Synthesis of 7-((3aR,4R,6R,6aS)-6-(((tert-butyldiphenylsilyl)oxy)methyl)-2,2-dimethyltetrahydrothieno[3,4-d][1,3]dioxol-4-yl)-2-chloro-5-((trimethylsilyl)ethynyl)-7H-pyrrolor2,3-d]pyrimidine-4-amine (Compound 45)

Compound 44 (72 mg, 0.101 mmol) was dissolved in tertiary butyl alcohol saturated with ammonia, and the reaction mixture was put into a stainless steel bomb and transferred under a condition in which it had been heated to 100°C in advance. The reaction mixture was stirred at the same temperature for 15 hours. The reaction mixture contained in the stainless steel bomb was cooled until the temperature reached room temperature, and then evaporated under reduced pressure conditions. The residue was purified by silica gel column chromatography to obtain Compound 45 (49.7 mg, 71%) as a colorless syrup.

¹H NMR (CDCl₃, 400 MHz): *δ* 7.69-7.65 (m, 4H), 7.45-7.35 (m, 6H), 7.28 (s, 1H), 6.40 (brs, 1H), 6.19-6.19 (m, 1H), 4.87-4.84 (m, 2H), 3.91-3.76 (m, 3H), 1.59 (s, 3H), 1.29 (s, 3H), 1.09 (s, 9H), 0.25 (s, 9H). HRMS (FAB) *m*/*z* [M + H]⁺ for C₃₅H₄₃ClN₄O₃SSi₂ found 691.2361.

### Preparation Example 9-5: Synthesis of ((3aS,4R,6R,6aR)-6-(4-amino-2-chloro-5-ethynyl-7H-pyrrolo[2,3-d]pyrimidin-7-yl)-2,2-dimethyltetrahydrothieno[3,4-d][1,3]dioxol-4-yl)methanol (Compound 46)

1 M tetra-n-butylammonium fluoride (0.217 mL, 0.217 mmol) and acetic acid (0.217 mmol) were added to Compound 45 (50.1 mg, 0.072 mmol) in anhydrous tetrahydrofuran, and the reaction mixture was stirred at room temperature for 2 hours. The reaction mixture was neutralized with a saturated aqueous NH₄Cl solution, distributed in ethyl acetate and water, and extracted three times with ethyl acetate. The organic solvent layer was dried over anhydrous MgSO₄, filtered, and then evaporated. The residue was purified by silica gel column chromatography to obtain Compound 46 (22.3 mg, 81%) as a yellow syrup.

¹H NMR (CDCl₃, 400 MHz): *δ* 7.30 (s, 1H), 6.29 (brs, 2H), 5.99-5.97 (m, 1H), 5.21-5.19 (m, 1H), 4.96-4.94 (m, 1H), 4.01-3.98 (m, 1H), 3.90-3.84 (m, 2H), 3.26 (s, 1H), 1.60 (s, 3H), 1.31 (s, 3H); LRMS (ESI) *m*/*z* [M + H]⁺ for C₁₆H₁₇ClN₄O₃S found 381.1100.

### Preparation Example 9-6: Synthesis of (2R,3R,4S,5R)-2-(4-amino-2-chloro-5-ethynyl-7H-pyrrolo[2,3-d]pyrimidin-7-yl)-5-(hydroxymethyl)tetrahydrothiophene-3,4-diol (Compound 14)

1 M boron trichloride (0.078 mL, 0.078 mmol) was added dropwise to Compound 46 (20.0 mg, 0.052 mmol) in dichloromethane at -78°C. The reaction mixture was stirred at the same temperature for 1 hour. The reaction mixture was neutralized with a phosphate buffer solution (pH 7.4), distributed in dichloromethane and water, and extracted three times with dichloromethane. The organic solvent layer was dried over anhydrous MgSO₄, filtered, and then evaporated. The residue was purified by silica gel column chromatography to obtain Compound 14 (14.8 mg, 83%) as a white solid.

¹H NMR (CDCl₃, 400 MHz): *δ* 7.82 (s, 1H), 6.05-6.03 (m, 1H), 4.45-4.42 (m, 1H), 4.25-4.23 (m, 1H), 3.89-3.80 (m, 2H), 3.73 (s, 1H), 3.52-3.44 (m, 1H); LRMS (ESI) *m*/*z* [M + H]⁺ for C₁₃H₁₃ClN₄O₃S found 341.0800.

### Experimental example 1: Evaluation of inhibitory activity against kinases

In order to examine the inhibitory activity of the nucleoside derivatives of the present invention synthesized in the Preparation Examples against kinases, the nucleoside derivatives were confirmed to have inhibitory activity against neurotrophic tyrosine kinase receptor type 1 or tropomyosin receptor kinase A (NTRK1), casein kinase 1 isoform delta (CSNK1D), dual specificity tyrosine-phosphorylation-regulated kinase 1A (DYRK1A), dual specificity tyrosine-phosphorylation-regulated kinase 1B (DYRK1B) and receptor-type tyrosine-protein kinase or fms like tyrosine kinase 3 or cluster of differentiation antigen 135 (CD135) or fetal liver kinase-2 (Flk2) (FLT3).

The inhibition measurement method is as follows. Most of the kinases were produced by culturing a phage in which BL21 *E. coli* was tagged with a kinase at 32°C until lysis, followed by centrifugation and filtration. Further, the kinases were produced using HEK-293 cells. Thereafter, the kinases were DNA tagged for qPCR. After an affinity resin was prepared by stirring a biotin-tagged substrate onto streptavidin-coated beads for 30 minutes, a non-specifically bound substrate was washed with a buffer (SeaBlock (Pierce), 1% BSA, 0.05% Tween 20, 1 mM DTT).

For a kinase test, the kinase, the substrate, the affinity resin, and a test material dissolved in dimethyl sulfoxide were stirred together in a buffer (20% SeaBlock, 0.17x PBS, 0.05% Tween20, and 6 mM DTT) at room temperature for 1 hour. Thereafter, an affinity substrate was washed with a buffer (1xPBS, 0.05% Tween20), and then suspended in an elution buffer (1xPBS, 0.05% Tween20, 0.5 µM non-biotinylated affinity ligand), and the resulting suspension was stirred. The concentration of the kinase was quantified by qPCR.

The following Table 9 shows the results of treating each kinase with 1 µM of Compounds 1a to 14, and (-) indicates no effect; (+) indicates weak inhibitory activity; (++) indicates a moderate level of inhibitory activity; (+++) indicates strong inhibitory activity; and ND indicates not determined.

**[Table 9]**

| Compound | NTRK1 | CSNK1D | DYRK1A | DYRK1B | FLT3 |
|---|---|---|---|---|---|
| 1a | +++ | ++ | +++ | +++ | ND |
| 1b | +++ | +++ | ++ | ++ | ND |
| 2a | +++ | ++ | ++ | ++ | ND |
| 2b | ++ | +++ | ++ | ++ | ND |
| 2c | - | - | - | - | ND |
| 2d | - | + | - | - | ND |
| 3 | ++ | ++ | +++ | +++ | ND |
| 4a | ++ | + | + | + | ND |
| 4b | + | + | + | + | ND |
| 4c | + | + | - | - | ND |
| 4d | ++ | + | - | - | ND |
| 5 | ++ | ++ | +++ | ++ | ND |
| 6 | + | ++ | +++ | ++ | ND |
| 7a | +++ | ++ | +++ | +++ | ND |
| 7b | +++ | ++ | ++ | ++ | ND |
| 7c | ++ | ++ | ++ | ++ | ND |
| 7d | ++ | ++ | ++ | ++ | ND |
| 7e | +++ | ++ | ++ | ++ | ND |
| 7f | +++ | ++ | ++ | ++ | ND |
| 7g | ++ | +++ | +++ | +++ | ND |
| 7h | ++ | +++ | +++ | +++ | ND |
| 8a | +++ | ++ | +++ | +++ | ND |
| 8b | +++ | ++ | +++ | +++ | ++ |
| 8c | +++ | ++ | +++ | ++ | ND |
| 8d | +++ | ++ | ++ | +++ | ND |
| 8e | ++ | ++ | ++ | ++ | ND |
| 9a | +++ | +++ | +++ | +++ | +++ |
| 9b | +++ | +++ | +++ | +++ | ND |
| 9c | +++ | +++ | +++ | +++ | ND |
| 10a | +++ | +++ | +++ | ++ | +++ |
| 10b | ++ | ++ | +++ | +++ | ND |
| 10c | ++ | ++ | ++ | ++ | ND |
| 11ai | +++ | +++ | +++ | ++ | +++ |
| 11aii | ++ | ++ | ++ | ++ | ND |
| 11aiii | ++ | ++ | ++ | ++ | ND |
| 11b | +++ | +++ | ++ | ++ | ND |
| 12a | ++ | ++ | +++ | ++ | ND |
| 12b | +++ | +++ | +++ | +++ | ND |
| 13a | ++ | ++ | ++ | ++ | ND |
| 13b | ++ | ++ | +++ | +++ | ND |
| 13c | +++ | +++ | ++ | ++ | ND |
| 13d | ++ | ++ | ++ | ++ | ND |
| 13e | ++ | ++ | ++ | ++ | ND |
| 13f | ++ | ++ | ++ | +++ | ND |
| 13g | +++ | +++ | ++ | +++ | ND |
| 14 | ++ | ++ | +++ | +++ | ND |

As shown in Table 9, it can be seen that the nucleoside derivatives of the present invention generally have excellent inhibitory activity against kinases such as NTRK1, CSNK1D, DYRK1A, DYRK1B, and FLT3.

### Experimental Example 2: Evaluation of inhibitory activity against cancer cells

In order to examine the anticancer activity of the nucleoside derivatives of the present invention synthesized in the Preparation Examples, the IC₅₀ of each of A549 (lung cancer cells), HCT116 (colon cancer cells), MDA-MB-231 (breast cancer cells), SK-Hep-1 (liver cancer cells), SNU638 (stomach cancer cells) and PC-3 (prostate cancer cells) against cancer cells was measured.

The test method is as follows. Cells were allowed to stand for 1 day and set as a control on day 0. Further, after a test material was added to the cells, the cells were allowed to stand for 3 days. Thereafter, the cells were stained with 1% acetic acid and 0.4% sulforhodamine B. The stained cells were dried and dissolved in 10 mM Tris (pH 10.0). Absorbance was measured at 515 nm, and the degree of cell division was determined using the following equation. After calculation as Cell division (%) = (average absorbance _{test material} - average absorbance _{day 0}) / (average absorbance _{control} - average absorbance _{day 0}) × 100, the degree of cell division was calculated through a non-linear regression analysis using Table Curve 2D v5.01 (Systat Software Inc., San Jose, CA, USA).

The results are shown in the following Table 10.

**[Table 10]**

| Compound | IC₅₀ (µM) | | | | | |
|---|---|---|---|---|---|---|
| | A549 | HCT116 | MDA-MB-231 | SK-HEP-1 | SNU638 | PC-3 |
| 1a | 1.02 | 2.16 | 3.31 | 3.38 | 1.95 | 2.46 |
| 1b | 1.65 | 1.74 | 2.72 | 1.38 | 2.41 | 2.48 |
| 2a | 0.97 | 0.56 | 0.31 | 0.22 | 0.47 | 0.20 |
| 2b | 7.57 | 6.35 | 8.83 | 7.81 | 6.32 | 9.21 |
| 2c | > 50 | > 50 | > 50 | > 50 | > 50 | > 50 |
| 2d | 41.62 | 30.57 | 30.16 | 10.34 | 27.47 | 15.43 |
| 3 | 0.06 | 0.03 | 0.05 | 0.05 | 0.03 | 0.004 |
| 4a | > 50 | > 50 | 45.51 | 6.05 | > 50 | > 50 |
| 4b | > 50 | > 50 | > 50 | > 50 | > 50 | > 50 |
| 4c | > 50 | > 50 | 29.57 | > 50 | > 50 | > 50 |
| 4d | > 50 | > 50 | 30.63 | 8.31 | > 50 | > 50 |
| 5 | 2.58 | 1.59 | 2.24 | 1.79 | 1.22 | 2.18 |
| 6 | 1.99 | 1.35 | 2.10 | 1.66 | 1.13 | 1.80 |
| 7a | 6.05 | 2.21 | 8.24 | 1.07 | 3.54 | > 50 |
| 7b | 3.19 | 2.86 | 2.13 | 0.57 | 1.31 | 2.05 |
| 7c | 0.27 | 0.23 | 0.29 | 0.18 | 0.13 | 0.23 |
| 7d | 0.35 | 0.30 | 0.36 | 0.25 | 0.19 | 0.33 |
| 7e | 6.47 | 6.42 | 4.68 | 4.39 | 4.84 | 5.59 |
| 7f | 0.070 | 0.038 | 0.074 | 0.050 | 0.048 | 0.078 |
| 7g | 2.58 | 3.60 | 1.56 | 5.62 | 3.78 | 2.96 |
| 7h | 4.78 | 1.61 | 6.79 | 5.09 | 5.23 | 3.56 |
| 8a | >50 | >50 | >50 | 1.15 | >50 | >50 |
| 8b | 28.56 | 26.31 | 32.39 | 21.25 | 23.34 | 29.12 |
| 8c | 10.07 | 13.72 | 10.36 | 12.21 | 12.22 | 14.75 |
| 8d | 11.16 | 8.90 | 9.34 | 13.83 | 23.70 | 6.67 |
| 8e | 45.16 | 12.45 | 6.78 | 10.01 | 9.34 | 4.65 |
| 9a | 0.17 | 0.37 | 0.42 | 0.26 | 0.33 | ND |
| 9b | 0.23 | 0.56 | 0.38 | 1.05 | 0.12 | 0.45 |
| 9c | 0.24 | 0.86 | 0.26 | 0.78 | 0.35 | 0.36 |
| 10a | 1.58 | 1.35 | 1.87 | 1.40 | 1.33 | 1.98 |
| 10b | 3.83 | 2.78 | 4.75 | 1.63 | 1.47 | 2.45 |
| 10c | 2.51 | 1.37 | 2.43 | 1.76 | 2.56 | 2.74 |
| 11ai | 4.59 | 2.77 | 4.11 | 3.31 | 2.45 | 3.54 |
| 11aii | 2.45 | 1.83 | 1.08 | 2.87 | 6.73 | 3.56 |
| 11aiii | 4.53 | 1.36 | 2.46 | 7.34 | 5.57 | 2.81 |
| 11b | 12.25 | 12.84 | 14.52 | 13.01 | 10.78 | 14.01 |
| 12a | 11.71 | 9.72 | 11.23 | 10.24 | 9.70 | 13.20 |
| 12b | 0.25 | 0.73 | 0.50 | 0.31 | 0.62 | ND |
| 13a | 0.59 | 0.85 | 1.15 | 1.27 | 1.20 | 1.39 |
| 13b | 0.96 | 0.86 | 1.32 | 0.64 | 1.31 | 1.74 |
| 13c | 2.91 | 3.38 | 5.16 | 2.74 | 4.12 | 7.01 |
| 13d | 6.83 | 4.31 | 5.93 | 7.81 | 5.66 | 9.90 |
| 13e | 4.33 | 2.84 | 5.33 | 5.11 | 3.54 | 5.34 |
| 13f | 2.36 | 6.37 | 2.41 | 5.78 | 9.35 | 2.45 |
| 13g | 5.27 | 9.73 | 5.67 | 2.13 | 8.46 | 2.24 |
| 14 | 0.35 | 7.28 | 0.52 | 1.37 | 5.46 | 2.45 |

As shown in Table 10, it can be seen that the nucleoside derivatives of the present invention generally exhibited low IC₅₀ values against cancer cells, such as A549, HCT116, MDA-MB-231, SK-Hep-1, SNU638, and PC-3, and thus, may be used as kinase inhibitors that can be expected to have anticancer effects.

## Claims

1. A nucleoside derivative represented by the following Chemical Formula 1 or a pharmaceutically acceptable salt thereof; 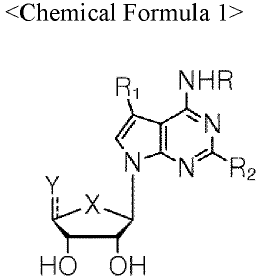 wherein, in Chemical Formula 1,
X is oxygen (O) or sulfur (S),
R is hydrogen (H); a substituted or unsubstituted C₁ to C₁₀ alkyl; or a substituted or unsubstituted C₆ to C₂₀ aryl or alkylaryl,
R₁ is a substituted or unsubstituted C₂ to C₁₀ heteroaryl; a substituted or unsubstituted C₂ to C₈ alkenyl; a substituted or unsubstituted C₆ to C₂₀ aryl; a substituted or unsubstituted C₂ to C₈ alkynyl; cyano; amide; or carboxyl,
R₂ is a halogen; substituted or unsubstituted amine; or a substituted or unsubstituted C₆ to C₂₀ aryl or alkylaryl, and
Y is hydrogen (H); an alkyl substituted with hydroxy, ester, alkoxy, aryl, heteroaryl, heterocycloalkyl, cycloalkyl, azide, amine, substituted amine, urea, or amide; or =CZ₂, and Z is each independently hydrogen (H) or a C₂ to C₈ alkyl.

2. The nucleoside derivative or the pharmaceutically acceptable salt thereof of claim 1, wherein:
the heteroaryl is furanyl, thiophenyl, pyrrolyl, pyranyl, pyrazolyl, pyridinyl, triazolyl, imidazolyl, thiazolyl, pyrimidinyl, pyrazinyl, pyridazinyl, indolyl, quinolinyl or purinyl,
the heterocycloalkyl is tetrahydrofuranyl, thiolanyl, pyrrolidinyl, tetrahydropyranyl, piperidinyl, dioxanyl, morpholino or tetrahydropyrimidinyl,
the aryl is optionally phenyl, naphthalenyl, anthracenyl or phenanthrenyl, and the alkylaryl is benzyl,
the alkynyl is ethynyl, propynyl, butynyl, pentynyl, hexynyl, heptynyl, octynyl, nonynyl or decynyl,
the alkenyl is ethenyl, propenyl, butenyl, pentenyl, hexenyl, heptenyl, octenyl, nonenyl or decenyl, and
the alkyl is methyl, ethyl, propyl, butyl or pentyl.

3. The nucleoside derivative or the pharmaceutically acceptable salt thereof of claim 1, wherein:
R₁ is furanyl, thiophenyl, vinyl, phenyl, thiomorpholino phenyl, thiomorpholino dioxide phenyl, sulfonamide phenyl, ethylsulfonamide phenyl, ethynyl, propynyl, butynyl, dimethylbutynyl, cyclopropylethynyl, cyano, amide or carboxy, and
R₂ is optionally chlorine (Cl) or amine (-NH₂).

4. The nucleoside derivative or the pharmaceutically acceptable salt thereof of claim 1, wherein:
Y is hydrogen, -CH₂OH, -CH₂OCOAr, -CH₂N₃, -CH₂NH₂, -CH₂NHCOAr, - CH₂NHCONH₂, -CH₂N-alkyl, -CH₂N-cycloalkyl, -CH₂N(CH₂CH₂)₂O or =CH₂, provided that
the 'Ar' is phenyl, chlorophenyl, methoxyphenyl, dimethylphenyl or nicotinyl, the 'alkyl' is methyl, ethyl, propyl, butyl or pentyl, and the 'cycloalkyl' is cyclopropyl, cyclobutyl, cyclopentyl or cyclohexyl.

5. The nucleoside derivative or the pharmaceutically acceptable salt thereof of claim 1, wherein:
Chemical Formula 1 is one of the following Chemical Formulae 1a and 1b, 2a to 2d, 3, 4a to 4d, 5, 6, 7a to 7h, 8a to 8e, 9a to 9c, 10a to 10c, 11ai to 11aiii, 11b, 12a and 12b, 13a to 13g and 14.

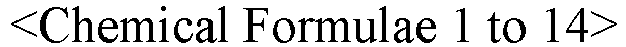

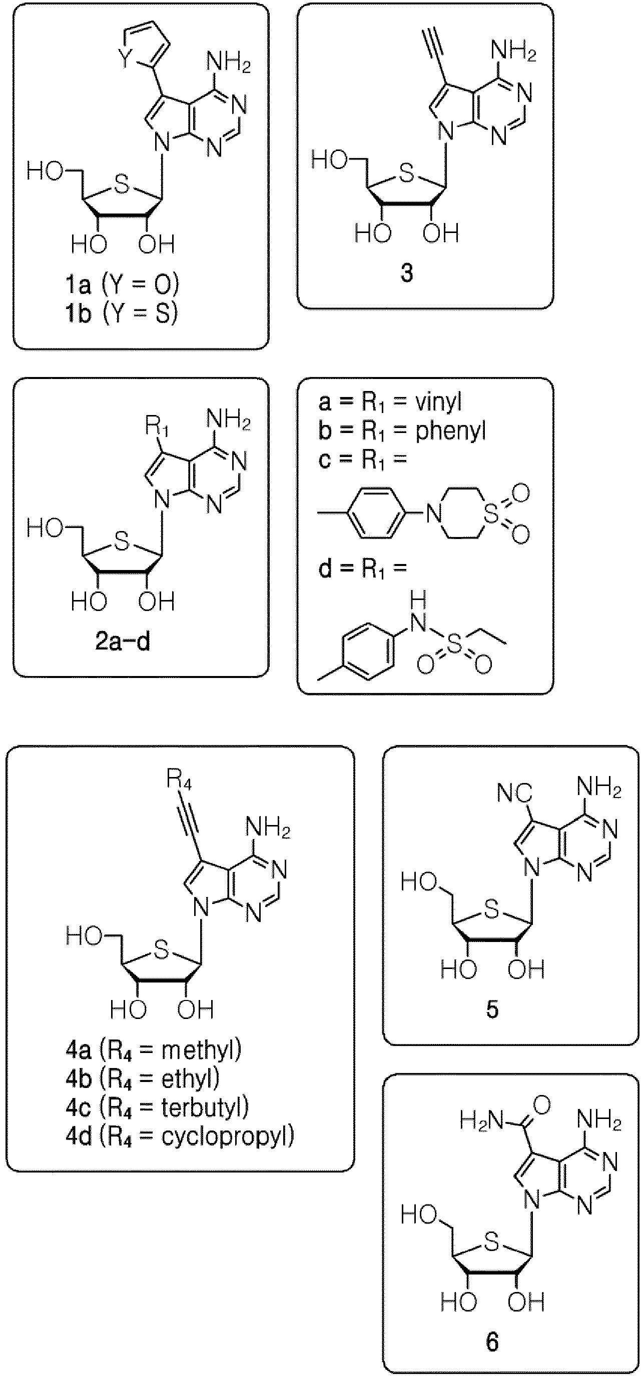

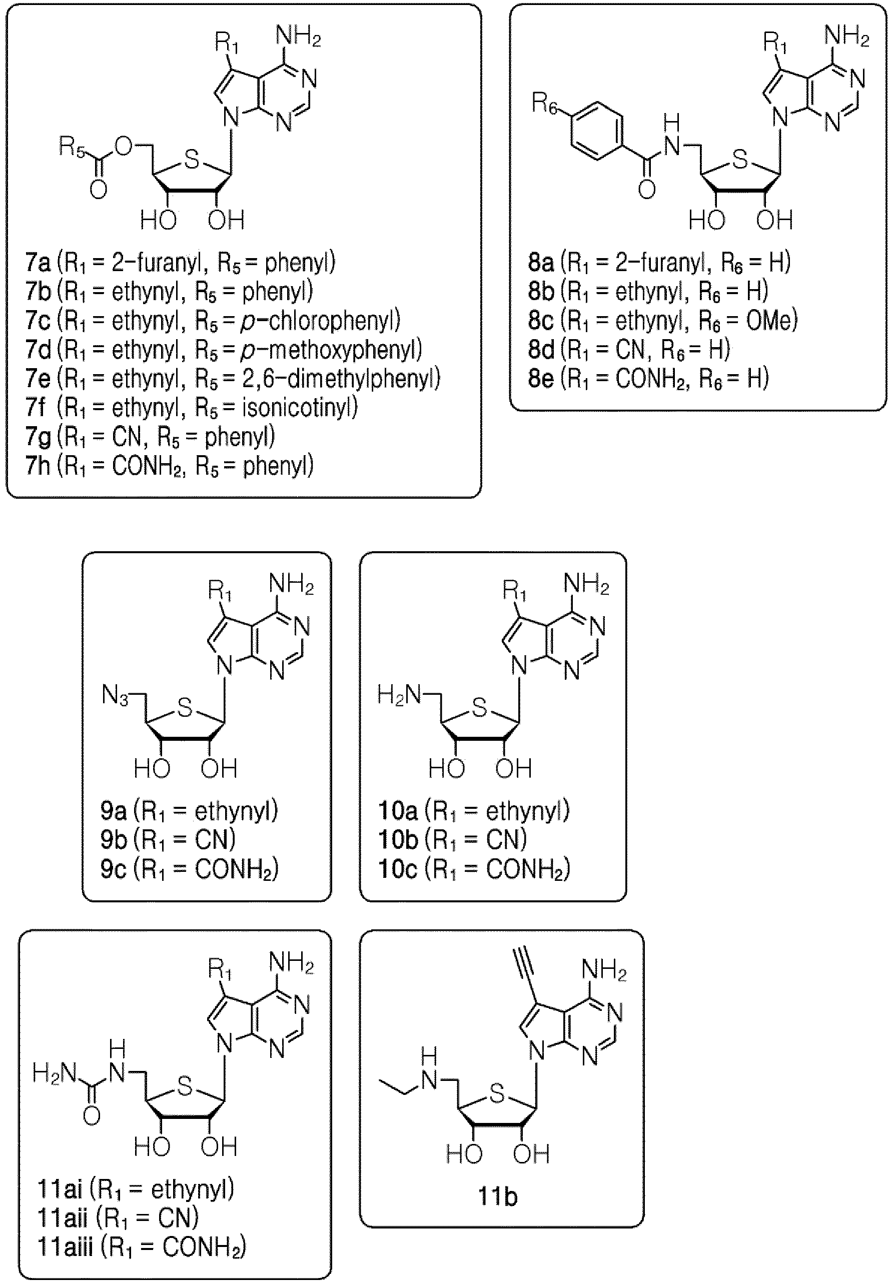

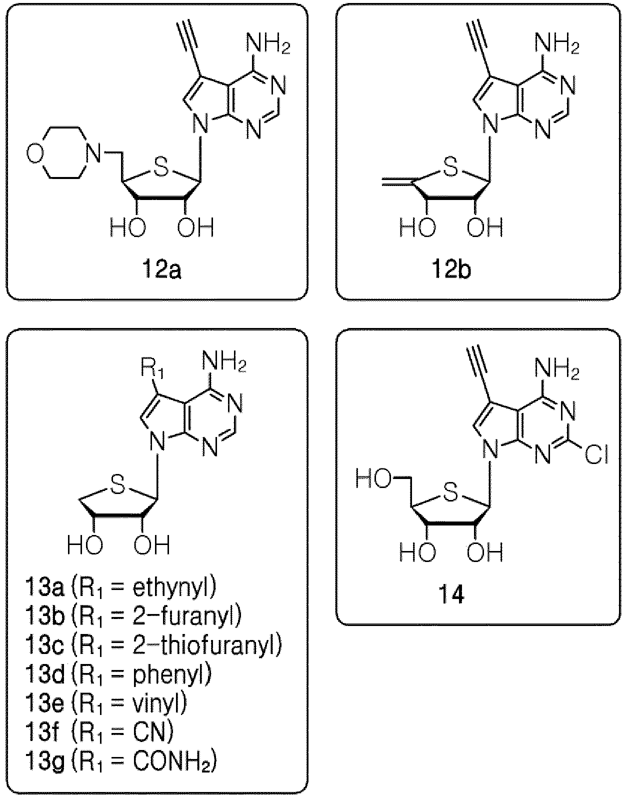

6. A pharmaceutical composition for preventing or treating cancer, comprising the nucleoside derivative or the pharmaceutically acceptable salt thereof of any one of claims 1 to 5.

7. The pharmaceutical composition of claim 6, wherein:
the cancer is one or more selected from the group consisting of lung cancer, colon cancer, breast cancer, liver cancer, stomach cancer, and prostate cancer.

8. The pharmaceutical composition of claim 6, wherein:
the nucleoside derivative or the pharmaceutically acceptable salt thereof has kinase inhibitory activity.
